# EUROPÄISCHE PATENTSCHRIFT

(11) **EP 2 046 304 B1**
(45) Veröffentlichungstag und Bekanntmachung des Hinweises auf die Patenterteilung: **09.10.2013**
(21) Anmeldenummer: 07727027.0
(22) Anmeldetag: 19.03.2007
(51) Int. Cl.: A61K 9/28, A61K 9/00, A61K 9/50

(54) **ARZNEIFORM MIT MEHRSCHICHTIGER TRENNSCHICHT**
PHARMACEUTICAL FORM WITH MULTILAYER SEPARATING LAYER
FORME PHARMACEUTIQUE AVEC COUCHE DE SEPARATION MULITCOUCHE

(30) Priorität: 27.07.2006 DE 102006035549
(43) Veröffentlichungstag der Anmeldung: 15.04.2009
(73) Patentinhaber: Evonik Röhm GmbH, 64293 Darmstadt (DE)
(72) Erfinder: LIZIO, Rosario, 64380 Rossdorf (DE); ROTH, Erna, 64297 Darmstadt (DE); PETEREIT, Hans-Ulrich, 64291 Darmstadt (DE)
(86) Internationale Anmeldenummer: PCT/EP2007/052549
(87) Internationale Veröffentlichungsnummer: WO 2008/012115

(56) Entgegenhaltungen:
- EP-A- 1 614 413
- EP-A- 1 728 512
- WO-A-2005/027890
- WO-A-2006/085335

## Beschreibung

### Gebiet der Erfindung

Die Erfindung betrifft eine Arzneiform mit **drei**schichtiger Trennschicht.

### Stand der Technik

EP 0 088 951 A2 beschreibt ein Verfahren zum Überziehen von Arzneiformen mittels eines in Wasser dispergierten Überzugsmittels. Für die Redispergierung von Carboxylgruppen-haltigen (Meth)acrylatcopolymeren von Pulvern zu Dispersionen wird die Teilneutralisation der Carboxylgruppen empfohlen. Die Salzbildung der sauren Gruppen tritt durch Umsetzung mit einer Base ein. Als Basen kommen Alkalien, wie z.B. Natronlauge, Kalilauge, Soda, Pottasche, Natriumbikarbonat, Trinatriumphosphat, Trinatriumcitrat oder Ammoniak oder physiologisch verträgliche Amine, wie Triethanolamin oder Tris-(hydroxymethyl)-aminomethan, in Betracht. Günstig in Bezug auf die Redispergierung ist ein Neutralisationsgrad von 0,1 bis 10 Gew.-% der im Copolymeren enthaltenen Carboxylgruppen.

WO 2004/096185 beschreibt eine Arzneiform und ein Verfahren zu ihrer Herstellung. Die Arzneiform ist mit einem anionischen (Meth)acrylatcopolymeren überzogen, das bei Bedarf teilneutralisiert werden kann. Um eine Lösung des anionischen Copolymers herzustellen, ist in der Regel eine teilweise oder vollständige Neutralisation der Säuregruppen nötig. Das anionische Copolymer kann z. B. nach und nach in einer Endkonzentration von 1 bis 40 Gew -% in Wasser eingerührt werden und dabei teilweise oder vollstandig neutralisiert werden durch Zugabe einer basischen Substanz wie z. B NaOH. KOH. Ammoniumhydroxyd oder organische Basen wie z. B Triethanolamin. Es ist auch möglich ein Pulver des Copolymeren einzusetzen, dem bereits bei seiner Herstellung zum Zweck der (Teil)neutralisation eine Base z B NaOH zugesetzt wurde, so dass das Pulver ein bereits (teil)neutralisiertes Polymer ist. Der pH-Wert der Lösung liegt in der Regel über 4, z. B Im Bereich von 4 bis ca. 7.

WO 2005/007139 beschreibt multipartikuläre Arzneiformen, enthaltend mucoadhaesiv formulierte Peptid- oder Proteinwirkstoffe. Die Anmeldung erwähnt, dass zwischen wirkstoffhaltiger und darmlöslicher Copolymer-Schicht eine trennende Schicht aufgebracht sein kann, die der Trennung von Wirkstoff und Überzugsmaterial zum Zwecke der Verhinderung von Interaktionen dient Diese Schicht kann aus inerten Filmbildnern (z.B. HPMC, HPC oder (Meth)acrylsäure-Copolymeren) oder z B Talkum oder einer anderen geeigneten pharmazeutischen Substanzen bestehen. Ebenso können Kombinationen aus Filmbildnern und Talkum oder ähnlichen Stoffen verwendet werden. Es ist auch möglich, eine Trennschicht aus teilweise bzw voll neutralisierten (Meth)acrylatcopolymer-Dispersionen aufzubringen. Die Trennschicht kann auch aus dem gleichen oder einem anderen mucoadhaesiven Polymer wie in der unterliegenden Matrixschicht bestehen Auf diese Weise kann eventuellen Interaktionen oder Unvertraglichkeiten des Wirkstoffs oder des mucoadhaesiven Polymers mit der filmbildenden (Meth)acrylatcopolymer-Schicht begegnet werden

WO 2005/027890 beschreibt Darreichungsformen mit einem Kern (a), einer darmsaftlöslichen Umhüllung und dazwischen eine zweischichtige Trennschicht aus filmbildenden inneren und wasserunlöslichen außeren Schicht.

### Aufgabe und Lösung

Während man bisher durchaus erfolgreich versuchte, bei darmsaftlöslich überzogenen Arzneiform eine hohe Reproduzierbarkeit der Wirkstoffabgabe zu erreichen, indem man beim Erreichen von spezifischen pH-Werten sich möglichst schnell auflösende Überzüge entwickelte, geht die vorliegende Erfindung von einem alternativen Konzept aus.

Die Erfinder haben festgestellt, dass bei Arzneiformen, die mit darmsaftlöslichen (Meth)acrylat-Copolymeruberzugen ausgestattet sind, permanente Interaktionen zwischen dem bereits austretendem Wirkstoff und gegebenenfalls vorhandenen dem Wirkstoff zugeordneten Substanzen und der sich noch in der Auflösung begriffenen Polymerschicht stattfinden Diese Interaktion wirkt grundsatzlich der Reproduzierbarkeit der Wirkstofffreisetzung entgegen

Anhand von raster-elektronenmikroskopischen Bildern von überzogenen Pellets, die aus künstlichem Darmsaft zu einem Zeitpunkt entnommen werden, bei dem die Wirkstoffabgabe bereits eingesetzt hat, kann beobachtet werden, dass sich die umgebende Polymerschicht meist noch nicht oder nur teilweise vom Kern der Arzneiform gelost hat Dieser Effekt beeinflusst die Wirkstoffabgabe da lokal ein Teil des Wirkstoffs mehr oder weniger ungehindert entweicht während andere Teile erst durch die Reste der umgebenden Hulle hindurch dringen müssen Da sich die Parameter dabei in praktisch nicht kontrollierter Weise permanent verandern, lauft dies einer optimal kontrollierten Wirkstoffabgabe mit hoher Reproduzierbarkeit entgegen Daher soll nach dem Konzept der vorliegenden Erfindung eine darmsaftlöslich überzogene Arzneiform bereitgestellt werden bei der sich die umgebende Polymerhülle zunächst zumindest annähernd vollständig auf- bzw. ablöst, bevor möglichst unmittelbar danach die Wirkstoffabgabe einsetzt.

Die Aufgabe wird gelöst durch eine Arzneiform, enthaltend einen wirkstoffhaltigen Kern, der mit einer Überzugsschicht aus einem magensaftresistenten, darmsaftlöslichen (Meth)acrylat-Copolymer umhüllt ist, wobei sich zwischen dem Kern und der Überzugsschicht eine Trennschicht, enthaltend ein filmbildendes wasserlösliches Polymer, befindet, dadurch gekennzeichnet, dass die Trennschicht dreischichtige ausgeführt ist, wobei eine innere Schicht mit einer wasserabweisenden Substanz vorhanden ist und zwei Schichten des filmbildenden wasserlöslichen Polymeren die Schicht mit der wasserabweisenden Substanz einschließen.

### Ausführung der Erfindung

### Wirkstoffhaltiger Kern

Die Arzneiform weist einen wirkstoffhaltigen Kern auf. Der Der Kern enthalt in der Regel 5 bis 100. bevorzugt 10 bis 50 Gew -% Wirkstoff sowie bis zu 95 bevorzugt 50 bis 90 Gew -% an weiteren pharmazeutischen Hilfsstoffen.

Wirkstoffhaltige Kerne können durch übliche Herstellungsverfahren wie direktes Verpressen. Verpressen von Trocken-. Feucht- oder Sintergranulaten Extrusion und anschließende Ausrundung feuchte oder trockene Granulation oder durch direkte Pelletierung (z.B. auf Tellern) oder durch Binden von Pulvern (Powder layering) auf wirkstofffreie Kugeln (Nonpareilles) oder wirkstoffhaltige Partikel z. B Wikrstoffkristalle erhalten werden

Als weitere pharmazeutische Hilfsstoffe können die Kerne neben dem Wirkstoff z B. enthalten: Bindemittel wie Zellulose und deren Derivate, Polyvinylpyrrolidon (PVP). Feuchthaltemittel. Zerfallsförderer, Gleitmittel, Sprengmittel. (Meth)acrylate. Starke und deren Derivate. Zucker Solubilisatoren oder andere pharmazeutisch ubliche Hilfsstoffe.

### Dreischichtige Trennschicht

Zwischen dem Kern und der Überzugsschicht befindet sich eine Trennschicht, die dreischichtig ausgeführt ist, wobei zwei Schichten eines filmbildenden, wasserlöslichen Polymeren eine Schicht mit einer wasserabweisenden Substanz einschließen.

In der Regel und für die Funktion ist es ausreichend, die Trennschicht dreischichtig auszuführen. Es ist prinzipiell auch möglich oder denkbar weitere Schichten, z. B. weitere Schichten des filmbildenden, wasserlöslichen Polymeren und darin eingeschlossene Schichten mit einer wasserabweisenden Substanz hinzufügen, ohne die Funktion der Trennschicht als solche zu beeinträchtigen.

Eine dreischichtige Trennschicht hat insbesondere die Funktion den Austritt des Wirkstoffs zu verlangsamen bis sich die äußere Polymerhülle aufgelöst bzw. abgelöst hat. Die zwischen zwei Schichten eines filmbildenden, wasserlöslichen Polymeren eingeschlossene Schicht mit einer wasserabweisenden Substanz trennt dabei, zumindest für eine gewisse Zeit den Wirkstoff von der äußeren wässrigen Umgebung ab, was zur angestrebten Verzögerung der Wirkstoffabgabe beitragen soll.

Der drei- oder gegebenenfalls mehr als dreischichtige Aufbau der Trennschicht hat den Vorteil, dass sich der Polymerüberzug bei mehr als 95 % der Pellets komplett auflöst, bevor die Wirkstofffreigabe leicht verzögert einsetzt. Die vollständige Auflösung des Polymerüberzugs kann anhand von rasterelektronenmikroskopischen Bildern (REM bzw. SEM) von Pellets nachgewiesen werden, bei denen die Wirkstofffreigabe im in-vitro-Freigabeversuch gerade eingesetzt hat. Bevorzugt sind zwei Schichten aus jeweils 3 - 15 Gew.-% HPMC mit einer dazwischen liegenden Schicht aus 3 - 15 Gew.-% Caprinsäure, jeweils bezogen auf das Gewicht des wirkstoffhaltigen Kerns.

### Filmbildendes, wasserlösliches Polymer

Das filmbildende, wasserlösliche Polymer ist bevorzugt besonders gut und schnell wasserlöslich, was die Auflösung bzw. die Ablösung der Überzugsschicht begünstigt. Die Wirkung der Schicht aus dem filmbildenden, wasserlöslichen Polymer ist daher auf diese kurzzeitige Wirkung ausgelegt

Das filmbildende, wasserlösliche Polymer kann nicht ionische Cellulosederivate, wie Hydroxypropylcellulose, Hydroxypropylmethylcellulose (HPMC), sowie Natrium-Carboxymethylcellulose, Polysaccharide, wie Stärke, Amylose, Alginat, Pektin, Xanthan sowie Gelatine, Polyethylenglykole und/oder Polyvinylpyrrolidon umfassen.

Das wasserlösliche Polymer kann besonders bevorzugt eine Hydroxypropylmethylcellulose mit einer Viskosität von 1 bis 20. bevorzugt 2 bis 10. besonders bevorzugt 4 bis 8 mPa s. bezogen auf eine 1 %-ige Lösung (Gewicht/Gewicht messbar z. B. nach Pharm.Eur 5.0. Methode 2.2 10, Rotationsviscosimeter) sein, geeignet ist z B. Methocel® E5.

Die Schichten des filmbildenden, wasserlöslichen Polymeren können jeweils 1 bis 50. bevorzugt 2 bis 12. bevorzugt 3 bis 8 Gew -% bezogen auf das Gewicht des wirkstoffhaltigen Kerns ausmachen

Das filmbildende, wasserlösliche Polymer kann eine Löslichkeit in demineralisiertem Wasser von mindestens 50 g/l bei 20 °C aufweisen (Wasserlöslichkeit in Anlehnung an Standardmethoden, wie z. B. Pharmeuropa - Technical Guide for the Elaboration of Monographs 3rd Edition (1999), Chapter IV. Appendix IV unter heftigem Schütteln für 1 min, 15 min stehen lassen bei 20 °C in gereinigtem Wasser).

### Wasserabweisende Substanz

Die wasserabweisende Substanz bildet bevorzugt eine unter physiologischen Bedingungen ab pH 5.5 sich rasch in Micellenform vom Kern ablosende Schicht. Die Ablösung in Micellenform erfolgt jedoch erst nach der Auflösung sich darüber befindenden Schicht mit dem filmbildenden, wasserlöslichen Polymer wenn der Kontakt zum ungebenden Medium hergestellt ist. Die Schicht mit der wasserabweisenden Substanz ist daher für eine kurzeitige Wirkung in der der Austritt des Wirkstoffs verlangsamt wird ausgelegt In der Regel ist die wasserabweisende Substanz kein Polymer

Die wasserabweisende Substanz kann insbesondere ein C₈- bis C₂₄-Fettalkohol, ein Ester von C₈- bis C₂₄- Fettalkoholen mit organischen Säuren, eine C₈- bis C₂₄-Fettsäure, wie z. B. Stearinsäure oder Caprinsäure, ein Ester von C₈- bis C₂₄- Fettsäuren mit Alkoholen oder Polyalkoholen, wie z. B. Glycerolmonostearat oder Glyceroldistearat, sein. Besonders bevorzugt sind Substanzen mit einem Schmelzpunkt nach DAB (Deutsches Arzneimittelbuch) im Bereich von 30 bis 40 °C.

Die Schicht mit der wasserabweisenden Substanz kann 0,1 bis 25, bevorzugt 1 bis 10, besonders bevorzugt 3 bis 8 Gew.-% bezogen auf das Gewicht des wirkstoffhaltigen Kerns ausmachen.

Die wasserabweisende Substanz kann eine Löslichkeit in Aceton von mindestens 50 g/l bei 20 °C aufweist (Löslichkeit in Aceton in Anlehnung an Standardmethoden, wie z. B. Pharmeuropa - Technical Guide for the Elaboration of Monographs, 3rd Edition (1999), Chapter IV, Appendix IV, unter heftigem Schütteln für 1 min, 15 min stehen lassen bei 20 °C in Aceton).

### Arzneiform mit einer dem Wirkstoff zugeordneten, die Verabreichung des Wirkstoffs fördernden Substanz

Die dreischichtige Trennschicht führt zu einem weiteren Aspekt der Erfindung.

Die Erfindung betrifft somit auch eine Arzneiform, umfassend einen Kern, sowie einen den Kern umhüllenden, magensaftresistenten, darmsaftlöslichen Polymerüberzug, wobei der Kern einen Wirkstoff und eine dem Wirkstoff zugeordnete, die Verabreichung des Wirkstoffs fördernde Substanz enthält, wobei sich zwischen dem Kern und der Überzugsschicht eine Trennschicht, enthaltend ein filmbildendes wasserlösliches Polymer, befindet, dadurch gekennzeichnet, dass eine dreischichtige Trennschicht vorhanden ist, die sich aus zwei Schichten eines wasserlöslichen, polymeren Filmbildners mit einer dazwischen liegenden Schicht aus einer hydrophoben Substanz zusammensetzt.

Unter "zugeordnet" ist zu verstehen, dass die Substanz, zur Förderung der Verabreichung des konkret enthaltenen Wirkstoffs bestimmt ist und daher zur Erreichung des angestrebten therapeutischen Effekts unverzichtbarer Bestanteil der Arzneiform ist. In der Regel liegt die Substanz in unmittelbarer Umgebung des Wirkstoffs vor und kann z.B. zusammen mit diesem in einer gemeinsamen Matrix, die gegebenenfalls noch weitere zugeordnete Substanzen oder weitere pharmazeutische Hilfsstoffe enthalten kann, eingebettet sein.

Die Erfindung kann besonders vorteilhaft angewendet werden, wenn der enthaltene Wirkstoff, ein Peptid, ein Protein, eine Nukleinsäure oder ein Polysaccharid, z. B. Heparin, oder ein Derivat der genannten Stoffklassen ist und mit einer zugeordneten, die Verabreichung des Wirkstoffs fördernden Substanz kombiniert ist (s. WO 2005/007139, WO 2006/061069). Gerade in diesen Fällen ist oftmals eine extrem hohe Reproduzierbarkeit der Wirkstoffabgabe gefordert (s. WO 2005/007139, WO 20061061069).

Die dem Wirkstoff zugeordnete Subtanz kann bevorzugt ein Penetrationsförderer und/oder oder ein mucoadhaesives Polymer sein. Die dem Wirkstoff zugeordnete Substanz, kann weiterhin eine Substanz sein, die den enzymatischen Abbau des Wirkstoffs durch in Verdauungstrakt vorkommende Enzyme inhibiert bzw hemmt. Die dem Wirkstoff zugeordnete Subtanz kann weiterhin auch ein Efflux-Pumpen-Inhibitor (Pgp-Inhibitor) sein

Beispiele für Penetrationsförderer sind Aminoalkyl(meth)acrlyat-Copoymere wie z. B Eudragit® E100 oder Eudragit® E PO (s. EP 1 302 201 A1). Geeignete Penetrationsförderer sind insbesondere Weichmacher wie beispielsweise Triethylcitrat, Acetyltrietylcitrat, Diethylsebacat, Dibutylsebacat, Polymere wie Carbomer Chitosan. Chitosan-Cystein, Natrium-Carboxymethylcellulose. N-Trimethyliertes Chitosan Polycarbophil-Cysteine, langkettige Fettsäuren, ihre Ester (beispielsweise Mono und Diglyceride) und ihre Salze wie Laurinsäure, Laurinsulfonsäure Palmitinsaure. Caprylsäure Capnnsaure Olsaure. Acylcarnitine, Chelatbildner wie EDTA, Salicylate, Cyclodextrine. Polyacrylsauren Gallensauren wie Cholsaure. Cholyltaurin. Cholylsarcosin, Chenodeoxycholsaure und ihre Salze wie Na-Cholat. Na-Glykocholat. Na-Taurocholat. Na-Taurodihydrofusidat, Na-Glykodihydrofusidat, Tenside und Emulgatoren wie insbesondere Polyethylen-660-12-Hydroxy-Stearat (Solutol® HS15) (Solutol HS15) Polysorbat 80 (Tween 80). Polyoxyethyliertes Kastoröl (Cremophor EL) Polyoxyethylene-polyoxypropylene glycol (Pluronic® F68), das Toxin Zonula Occludens Toxin (ZOT) sowie Vitamine wie Vitamin E (Tocopherol) und dessen Derivate oder Vitamin B12.

Beispiele für Polymere mit mucoadhaesiver Wirkung sind insbesondere Chitosane (Chitosan und Derivate Chitosane), (Meth)acrylatcopolymere, bestehend aus 20 - 45 Gew -% Methylmethacrylat und 55 bis 80 Gew -% Methacrylsaure Cellulosen mit mucoadhaesiver Wirkung, insbesondere Methylcellulosen wie Na-Carboxymethylcellulose (z B Blanose®).

Beispiele für Enzyminhibitoren sind der Bowman Birk Inhibitor (s. US 2004/0219216 A1) Zusatze von Sauren (EP 0 929 270 B1, US 6.086.918) oder Aminoalkyl(meth)acrlyat-Copoymere wie z. B Eudragit® E100 oder Eudragit® E PO (s EP 1 466 626 A1) Pharmazeutisch geeignete Proteaseinhibitoren sind beispielsweise Antipain, Aprotinin, Bacitracin, Benzamidin, Bestatin, Captopril, Chymostatin, Chicken Ovoinhibitor, EDTA-Na₂, Chitosan-EDTA-conjugates, Naglycocholate. Leupeptin, Pepstatin. Soybean trypsin inhibitors, Thiorphan, Tos-Lys-Chloromethylketon. Potato carboxypeptidase Inhibitor

Beispiele für Efflux-Pumpen-Inhibitoren sind z. B Ketokonazol oder Polyethylen-660-12-Hydroxy-Stearat (Solutol® HS15)

Anhand von rasterelektronenmikroskopischen Bildern (REM bzw. SEM) von Pellets bei denen die Wirkstofffreigabe im in-vitro-Freigabeversuch gerade eingesetzt hat kann festgestellt werden, dass die erfindungsgemaße dreischichtige Trennschicht recht zuverlassig bewirkt, dass sich die Überzugsschicht zu diesem Zeitpunkt bereits nahezu immer vollständig vom Kern getrennt hat bzw aufgelöst ist.

Die mindestens zweischichtige Trennschicht bewirkt insbesondere, dass erfindungsgemäße Arzneiformen in Form von anfänglich überzogenen Pellets, die zum Zeitpunkt zwischen der 10 und 30 %-igen Wirkstoffabgabe aus einem in-vitro Wirkstofffreisetzungsversuch nach USP entnommen werden, in rasterelektronenmikroskopischen Bildern bei einer Stichprobe von 100 Pellets zu mindestens 95 % bevorzugt zu mindestens 98 %, keine deutlich erkennbaren Reste der Polymerhülle mehr aufweisen

Dies verhindert dass aufgrund von verbleibenden Resten des Überzugs eine unkontrollierte vorzeitige Trennung des Wirkstoffs von mit dem Wirkstoff formulierten und auf den Wirkstoff abgestimmten penetrationsfördernden, mucoadhaesiven und/oder enzyminhibierenden Substanz eintritt. Dies kann nämlich dann geschehen, wenn eine der Komponenten durch partielle lokal beschränkte Offnungen des Polymerüberzugs bevorzugt entweicht, während die andere Komponente noch zurückbleibt. Erfindungsgemäß wird so mit höherer Sicherheit erreicht, dass der Wirkstoff und die zugehörige penetrationsfördernde, mucoadhaesive und/oder enzyminhibierende Substanz den Wirkort wie beabsichtigt gleichzeitig erreichen

Diese vorteilhafte Wirkung ist nicht auf bestimmte Wirkstoffe beschrankt, sofern eine zugeordnete Subtanz vorhanden ist, die dazu bestimmt ist die Verabreichung des konkret enthaltenen Wirkstoffs zu fördern und auf diese Weise die therapeutische Wirksamkeit der Arzneiform sicherzustellen Die mindestens zwei- oder dreischichtige Trennschicht dient dazu, auch geringfügige vorzeitige und unerwunschte Entmischungen oder Trennungen des Wirkstoffs und seiner zugeordneten Substanz sicherzustellen.

Bei schwerlöslichen Wirkstoffen und insbesondere bei Wirkstoffen, die ein Peptid ein Protein, eine Nukleinsäure oder ein Polysaccharid oder ein Derivat der genannten Stoffklassen sind, ist die Formulierung mit penetrationsfördernden und/oder einer mucoadhaesiven Substanzen jedoch besonders kritisch, so dass die Erfindung bevorzugt für diese Wirkstoffe angewandt werden kann Insbesondere kann dieser Aspekt der Erfindung auch angewandt werden wenn die genannten Wirkstoffklassen mit einer den enzymatischen Abbau des Wirkstoffs inhibierende Substanz kombiniert sind weil hier der therapeutische Effekt in besonders kritischer Weise davon abhangt dass der Wirkstoff bis zu seinem Auftreffen auf den Wirkort vor enzymatischem Abbau geschutzt wird

### Magensaftresistente, darmsaftlösliche (Meth)acrylat-Copolymere

Die erfindungsgemäße Arzneiform weist einen Überzug aus einem magensaftresistenten, darmsaftloslichen (Meth)acrylat-Copolymer auf Geeignet sind anionische (Meth)acrylat-Copolymere

Das anionische (Meth)acrylat-Copolymer kann zu 25 bis 95. bevorzugt zu 40 bis 95. insbesondere zu 60 bis 40 Gew. -% aus radikalisch polymerisierten C₁-bis C₄-Alkylestern der Acryl- oder der Methacrylsaure und zu 75 bis 5, bevorzugt 60 bis 5, insbesondere 40 bis 60 Gew -% aus (Meth)acrylatMonomeren mit einer anionischen Gruppe zusammengesetzt sein.

In der Regel addieren sich die genannten Anteile zu 100 Gew -%. Es können jedoch zusatzlich, ohne daß dies zu einer Beeinträchtigung oder Veränderung der wesentlichen Eigenschaften fuhrt geringe Mengen im Bereich von 0 bis 10, z B 1 bis 5 Gew.-% weiterer vinylisch copolymerisierbarer Monomere, wie z B Hydroxyethylmethacrylat oder Hydroxyethylacrylat enthalten sein. Bevorzugt sind keine weiteren vinylisch copolymerisierbaren Monomere enthalten.

C₁- bis C₄-Alkylestern der Acryl- oder Methacrylsaure sind insbesondere Methylmethacrylat Ethylmethacrylat. Butylmethacrylat Methylacrylat. Ethylacrylat und Butylacrylat

Ein (Meth)acrylat-Monomer mit einer anionischen Gruppe ist z B. Acrylsäure bevorzugt ist Methacrylsaure

Geeignet sind anionische (Meth)acrylat Copolymere aus 40 bis 60, Gew.-% Methacrylsaure und 60 bis 40 Gew -% Methylmethacrylat oder 60 bis 40 Gew.-% Ethylacrylat (Typen EUDRAGIT® L oder EUDRAGIT® L100-55) EUDRAGIT® L ist ein Copolymer aus 50 Gew -% Methylmethacrylat und 50 Gew -% Methacrylsaure. Der pH-Wert des Beginns der spezifischen Wirkstofffreisetzung in Darmsaft oder künstlichem Darmsaft kann mit pH 6.0 angegeben werden

EUDRAGIT® L100-55 ist ein Copolymer aus 50 Gew -% Ethylacrylat und 50 Gew -% Methacrylsaure EUDRAGIT® L 30 D-55 ist eine Dispersion enthaltend 30 Gew -% EUDRAGIT® L 100-55 Der pH-Wert des Beginns der spezifischen Wirkstofffreisetzung in Darmsaft oder künstlichem Darmsaft kann mit pH 5.5 angegeben werden

Ebenso geeignet sind anionische (Meth)acrylat Copolymere aus 20 bis 40 Gew -% Methacrylsaure und 80 bis 60 Gew -% Methylmethacrylat (Typ EUDRAGIT® S) Der pH-Wert des Beginns der spezifischen Wirkstofffreisetzung in Darmsaft oder kunstlichem Darmsaft kann mit pH 7.0 angegeben werden.

Geeignet sind (Meth)acrylat Copolymere, bestehend aus 10 bis 30 Gew.-% Methylmethacrylat 50 bis 70 Gew -% Methylacrylat und 5 bis 15 Gew -% Methacrylsaure (Typ EUDRAGIT® FS) Der pH-Wert des Begmns der spezifischen Wirkstofffreisetzung in Darmsaft oder künstlichem Darmsaft kann mit pH 7.0 angegeben werden

EUDRAGIT® FS ist ein Copolymer aus 25 Gew -% Methylmethacrylat. 65 Gew.-% Methylacrylat und 10 Gew -% Methacrylsaure EUDRAGIT® FS 30 D ist eine Dispersion enthaltend 30 Gew.-% EUDRAGIT® FS

Weiterhin geeignet ist ein Copolymer, welches sich aus
20 bis 34 Gew -% Methacrylsaure und/oder Acrylsäure,
20 bis 69 Gew.-% Methylacrylat und
0 bis 40 Gew.-% Ethylacrylat und/oder gegebenenfalls
0 bis 10 Gew.-% weiteren vinylisch copolymerisierbarer Monomeren
zusammensetzt, mit der Maßgabe daß die Glastemperatur des Copolymers nach ISO 11357-2. Punkt 3.3.3, höchstens 60 °C betragt. Dieses (Meth)acrylatcopolymer ist wegen seiner guten Reißdehungseigenschaften insbesondere zum Verpressen von Pellets zu Tabletten geeignet

Weiterhin geeignet sind Copolymere aus
20 bis 33 Gew -% Methacrylsaure und/oder Acrylsäure,
5 bis 30 Gew.-% Methylacrylat und
20 bis 40 Gew.-% Ethylacrylat und
größer 10 bis 30 Gew -% Butylmethacrylat und
gegebenenfalls
0 bis 10 Gew -% weiteren vinylisch copolymerisierbarer Monomeren, wobei sich die Anteile der Monomeren zu 100 Gew -% addieren,
zusammensetzt, mit der Maßgabe, daß die Glastemperatur des Copolymers (glass transition temperature) nach ISO 11357-2. Punkt 3 3 3 (midpoint temperature *T*_{mg}) 55 bis 70 °C betragt Copolymere dieses Typs sind wegen seiner guten mechanischen Eigenschaften insbesondere zum Verpressen von Pellets zu Tabletten geeignet

Das oben genannte Copolymer setzt sich insbesondere zusammen aus radikalisch polymerisierten Einheiten von
20 bis 33. bevorzugt 25 bis 32. besonders bevorzugt 28 bis 31 Gew -% Methacrylsaure oder Acrylsäure bevorzugt ist Methacrylsäure,
5 bis 30. bevorzugt 10 bis 28. besonders bevorzugt 15 bis 25 Gew.-% Methylacrylat.
20 bis 40 bevorzugt 25 bis 35. besonders bevorzugt 18 bis 22 Gew -% Ethylacrylat, sowie
größer 10 bis 30 bevorzugt 15 bis 25. besonders bevorzugt 18 bis 22 Gew -% Butylmethacrylat
zusammen, wobei die-Monomerzusammensetzung so gewählt wird, daß die Glastemperatur des Copolymers 55 bis 70 °C, bevorzugt 59 bis 66. besonders bevorzugt 60 bis 65 °C betragt

Unter Glastemperatur wird hier insbesondere die midpoint temperature *T*_{mg} nach ISO 11357-2. Punkt 3.3.3, verstanden. Die Messung erfolgt ohne Weichmacherzusatz, bei Restmonomergehalten (REMO) von weniger als 100 ppm, bei einer Aufheizrate von 10 °C/min und unter Stickstoffatmosphäre.

Das Copolymer besteht bevorzugt in wesentlichen bis ausschließlich, zu 90, 95 oder 99 bis 100 Gew -% aus den Monomeren Methacrylsaure. Methylacrylat Ethylacrylat und Butylmethacrylat in den oben angegebenen Mengenbereichen

Es können jedoch zusatzlich, ohne daß dies zu einer Beeinträchtigung der wesentlichen Eigenschaften fuhren muß geringe Mengen im Bereich von 0 bis 10 z. B 1 bis 5 Gew -% weiterer vinylisch copolymensierbarer Monomere, wie z B. Methylmethacrylat. Butylacrylat. Hydroxyethylmethacrylat. Vinylpyrrolidon, Vinylmalonsäure, Styrol, Vinylalkohol, Vinylacetat und/oder deren Derivate enthalten sein

### Herstellung der anionischen (Meth)acrylatcopolymere

Die Herstellung des anionischen (Meth)acrylatcopolymere kann in an sich bekannter Weise durch radikalische Polymerisation der Monomeren erfolgen (siehe z B EP 0 704 207 A2 und EP 0 704 208 A2). Das erfindungsgemäße Copolymer ist in an sich bekannter Weise durch radikalische Emulsionspolymerisation in wäßriger Phase in Gegenwart von vorzugsweise anionischen Emulgatoren herstellbar, beispielsweise nach dem in DE-C 2 135 073 beschriebenen Verfahren

Das Copolymerisat kann nach gangigen Verfahren der radikalischen Polymerisation kontinuierlich oder diskontinuierlich (Batch-Verfahren) in Gegenwart radikalbildender Initiatoren und gegebenenfalls Reglern zur Einstellung des Molekulargewicht in Substanz, in Lösung, durch Perlpolymerisation oder in Emulsion hergestellt werden Das mittlere Molekulargewicht Mw (Gewichtsmittel bestimmt z B durch Messung der Lösungsviskosität) kann z B im Bereich von 80.000 bis 1.000.000 (g/mol) liegen Bevorzugt ist die Emulsionspolymerisation in wäßriger Phase in Gegenwart wasserloslicher Initiatoren und (vorzugsweise anionischer) Emulgatoren

Im Falle der Substanzpolymerisation kann das Copolymer in fester Form durch Brechen, Extrusion, Granulieren oder Heißabschlag erhalten werden.

Die (Meth)acrylatcopolymere werden in an sich bekannter Weise durch radikalische Substanz-, Lösungs-, Perl- oder Emulsionspolymerisation erhalten. Sie müssen vor der Verarbeitung durch geeignete Mahl-, Trocken- oder Sprühprozesse in den erfindungsgemäßen Teilchengrößenbereich gebracht werden. Dies kann durch einfaches Brechen extrudierter und abgekühlter Granulatstränge oder Heißabschlag erfolgen.

Insbesondere bei Mischung mit weiteren Pulvern oder Flüssigkeiten kann der Einsatz von Pulvern vorteilhaft sein. Geeignete Gerätschaften zur Herstellung der Pulver sind dem Fachmann geläufig, z. B. Luftstrahlmühlen, Stiftmühlen, Fächermühlen. Gegebenenfalls können entsprechende Siebungsschritte einbezogen werden. Eine geeignete Mühle für industrielle Großmengen ist zum Beispiel eine Gegenstrahlmühle (Multi Nr. 4200), die mit ca. 6 bar Überdruck betrieben wird.

### Teilneutralisation

In einer bevorzugten Ausfuhrungsform wird der (Meth)acrylat-Copolymer-Überzug der erfindungsgemäßen Arzneiform in teilneutralisierter Form eingesetzt. Die Teilneutralisation beschleunigt die Auflösung bzw. Ablösung der Überzugsschicht von der Arzneiform Dadurch wird das Zusammenspiel von Auflösung bzw Ablösung der Überzugsschicht beim spezifischen pH-Wert in einem ersten Schritt und der sich erst dann unmittelbar anschließenden Wirkstofffreisetzung begünstigt

Das anionische (Meth)acrylat-Copolymer des Überzugs kann insgesamt oder anteilig mittels einer Base in Summe teilneutralisiert sein Wenn keine Mischung vorliegt, ist das vorhandene (Meth)acrylat-Copolymere einheitlich teilneutralisiert Im Falle von Mischungen kann nicht neutralisertes (Meth)acrylat-Copolymeren im Gemisch mit vollständig und/oder teilneutralisiertem (Meth)acrylat-Copolymeren vorliegen Gegebenenfalls können auch Mischungen von (Meth)acrylat-Copolymere unterschiedlichen Neutralisations- bzw Teilneutralisations-graden vorliegen

Die anionischen Gruppen des insgesamt vorhandenen (Meth)acrylat-Copolymers sollen dabei bevorzugter Weise in Summe, d h. gegebenenfalls im rechnerischen Mittel zu 0.1 bis 25 besonders bevorzugt zu 5 bis 15 % neutralisiert sein

Es ist bekannt anionische (Meth)acrylat-Copolymere in teilneutralisierter Form einzusetzen Dadurch wird eine verbesserte Löslichkeit des Polymeren in Wasser und eine Stabilisierung der Polymerdispersionen erreicht Als Basen für die Teilneutralisation werden in der Regel Substanzen wie NaOH, KOH. Ammoniumhydroxyd oder organische Basen, wie z. B. Triethanolamin. angegeben (s. z. B. EP 0 088 951 A2 oder WO 2004/096185).

Vergleicht man z. B mittels NaOH teilneutralisierte und nicht teilneutralisierte Filme aus anionischen (Meth)acrylatcopolymeren, so stellt man fest, dass sich die teilneutralisierten Filme schneller in einen Puffersystem bei ihrem spezifischen Auflösungs-pH-Wert auflosen als die nicht neutralisierten Filme

Bisher nicht bekannt ist der folgende Effekt Die Erfinder haben festgestellt, dass sich das oben beschriebene Verhalten teilneutralisierter Filme und mit teilneutralisierten Filmen uberzogener Arzneiformen nur vermindert erstellt, wenn Basen, die aus in EP 0 088 951 A2 oder WO 2004/096185 bekannt sind (z. B NaOH) für die Teilneutralisation einsetzt, wenn man die Filme oder Arzneiformen zunächst bei pH 1.2 für 2 Stunden belasst, bevor man auf den spezifischen pH-Wert des Beginns der Wirkstofffreisetzung umpuffert Gerade diese Bedingungen liegen jedoch in-vivo vor, wenn eine Arzneiform zunächst in den Magen gelangt und dann erst in den Darmtrakt befördert wird Die oben erwahnte Teilneutralisation von anionischen (Meth)acrylatcopolymeren ist daher nur bedingt geeignet um eine beschleunigtes Wirkstofffreisetzungsverhalten zu erzeugen

Ein verbesserter Beschleunigungseffekt in-vivo kann erreicht werden, wenn für die Teilneutralisation mit Lysin (M_{w} 146) oder eine kationische, organische Basen mit einem M_{w} > 150. bevorzugt > 155 besonders bevorzugt > 160. z. B. von > 150 bis 20 000 eingesetzt wird Insbesondere geeignet sind Lysin oder die kationischen basischen Aminosauren Histidin Arginin Die Aminosauren Glutamin und Asparagin sind kaum oder nicht geeignet da sie eine nicht protonierte Saureamidfunktion aufweisen und somit nicht den kationischen Basen zuzurechnen sind

Geeignet für die Teilneutralisation können weiterhin natürliche oder synthetische Oligomere oder Polymere sein, z. B. aus 3 bis 100 bevorzugt 5 bis 25 Einheiten, von Histidin, Arginin oder Lysin, Poly-Histidine, Poly-Arginine, Poly-Lysine, kationische bzw zwitterionische Phopholipide, wie z. B. Phosphatidylcholin,

Geeignet für die Teilneutralisation können weiterhin Ribonukleoside sein Kondensationsprodukte der Hydroxylfunktion am Kohlenstoffatom 1 der Ribose mit der heterocyklischen Aminofunktion der Basen Adenin. Guanin. Cytosin. Thymin oder Uracil entsprechend dem Vorkommen in der RNA

Geeignet für die Teilneutralisation können weiterhin Desoxyribonukleoside sein Kondensationsprodukte der Hydroxylfunktion am Kohlenstoffatom 1 der Desoxyribose mit der heterocyclischen Aminofunktion der Basen Adenin Guanin. Cytosin, Thymin oder Uracil entsprechend dem Vorkommen in der DNA

Geeignet für die Teilneutralisation können weiterhin Basen aus kationischen oberflachenaktiven Hilfsstoffen oder Emulgatoren sein, wie Benzalkonium ( CAS RN 8001-54-5), Benzethonium (CAS 121-54-0), Cetalkonium (CAS 122-18-9). Cetrimide ( CAS 8044-71-1), Cetrimonium ( CAS 57-09-0), Cetylpyridinium (CAS 123-03-5) Stearalkonium ( CAS 122-19-0), Diallyldimethylammonium ( CAS 230-993-8)

Bedingt geeignet für die Zwecke der Erfindung sind Basen die in EP 0 088 951 A2 oder WO 2004/096185 genannt sind Insbesondere Natronlauge Kalilauge (KOH) Ammoniumhydroxyd oder organische Basen wie z. B. Triethanolamin, Soda. Pottasche, Natnumbikarbonat Trinatriumphosphat, Trinatriumcitrat oder Ammoniak oder physiologisch vertragliche Amine, wie Triethanolamin oder Tris-(hydroxymethyl)-aminomethan.

Diese Basen weisen ein Mw von höchstens 150 auf (Triethanolamin). Obwohl Triethanolamin mit seinem Molekulargewicht nahe bei den Aminosauren Histidin. Arginin. Lysin liegt, tritt der auflosungsbeschleunigende Effekt mit dieser Substanz in-vivo nur geringem Maße ein. Trinatriumphosphat, Tnnatnumcitrat sind nicht kationischer Natur, sondern Salze der entsprechenden Sauren Ammoniumhydroxyd, Natronlauge. Kalilauge (KOH). Soda. Pottasche. Natriumbikarbonat weisen nur geringe Molekulargewichte auf bzw. sind den anorganischen Basen zuzurechnen

Bevorzugt enthalt der Polymeruberzug Lysin oder Arginin oder Arginin und Lysin als Teilneutralisationsmittel

Besonders bevorzugt enthalt der Polymeruberzug Lysin in einer Konzentration von 10 bis 30 Gew -%, bezogen auf die Trockensubstanz des Polymers

Insbesondere kann der Polymerüberzug Lysin oder Arginin oder Arginin und Lysin als Neutralisationsmittel in Kombination mit 5 bis 25. bevorzugt 8 bis 20 Gew.-% eines Weichmachers bezogen auf das Polymer enthalten

Das Molekulargewicht der genannten Substanzen ist bekannt bzw kann anhand der in Molekül vorhandenen Atome anhand der Atomgewichte errechnet werden

### Einstellung des Teilneutralisationsgrades durch Mischungen

Verfahrenstechnische Vorteile bei der Einstellung des Teilneutralisationsgrades können sich auch durch die bereits oben erwähnten Mischungen ergeben.

Es ist beispielsweise möglich, ein nicht teilneutralisiertes, anionisches (Meth)acrylat-Copolymer, bestehend aus radikalisch polymerisierten Einheiten von 25 bis 95 Gew.-% C₁- bis C₄-Alkylestern der Acryl- oder der Methacrylsäure und 5 bis 75 Gew.-% (Meth)acrylat-Monomeren mit einer anionischen Gruppe mit einem teilneutralisierten (Meth)acrylatcopolymeren gleicher Monomerzusammensetzung zu mischen, so dass bevorzugter Weise im rechnerischen Durchschnitt der Mischung 0.1 bis 25 % der enthaltenen anionischen Gruppen neutralisiert sind.

Die Mischung kann z. B. hergestellt werden, indem in eine Dispersion eines nicht teilneutralisierten, anionischen (Meth)acrylat-Copolymeren ein Pulver eingerührt wird, das aus einer Dispersion eines teilneutralisierten, anionischen (Meth)acrylat-Copolymeren. z. B. durch Sprüh- oder Gefriertrocknung, gewonnen wurde.

### Mischungen

Das den Kern umhüllende, magensaftresistente, darmsaftlösliche und gegebenenfalls teilneutralisierte (Meth)acryfat-Copolymer wird in der Regel ohne Beimengung weiterer Polymere eingesetzt. Das (Meth)acrylat-Copolymer eignet sich jedoch auch zur Mischung mit anderen pharmazeutisch genutzten Copolymeren, um die Eigenschaften zu modifizieren. Mischungen erhöhen die Gestaltungsfreiraume des Fachmanns bei der Einstellung speziell modifizierter Freigabeprofile.

Es können daher Mischung des den Kern umhüllenden, magensaftresistenten, darmsaftlöslichen und gegebenenfalls teilneutralisierte (Meth)acrylat-Copolymer vorliegen mit:

Copolymeren aus Methylmethacrylat und/oder Ethylacrylat und gegebenenfalls weniger als 5 Gew -% Methacrylsaure, mit Copolymeren aus Methylmethacrylat. Butylmethacrylat und Dimethylethylmethacrylat, mit Copolymeren aus Methylmethacrylat Ethylacrylat und Trimethylammoniumethylmethacrylat, Polyvinylpyrolidonen (PVP), Polyvinylalkoholen Polyvinylalkohol-Polyethylenglycol-Graft-Copolymeren (Kollicoat®) Starke und deren Derivaten, Polyvinylacetatphtalat (PVAP, Coateric®), Polyvinylacetat (PVAc, Kollicoat), Vinylacetat-Vinylpyrolidon-Copolymer (Kollidon® VA64), Vinylacetat : Crotonsaure-Copolymer 9:1 (VAC : CRA. Kollicoat® VAC) Polyethylenglykolen mit einem Molekulargewicht über 1000 (g/mol) Chitosan, einer vernetzten und/oder unvernetzten Polyacrylsaure, einem Na-Alginat, und/oder ein Pektin

Bevorzugt betragt der Anteil des magensaftresistenten, darmsaftlöslichen (Meth)acrylat-Copolymeren in der Mischung mindestens 50 Gew -%, besonders bevorzugt mindestens 75 Gew -%, insbesondere mindestens 90 oder bevorzugt mindestens 95 Gew -%, so dass dessen Eigenschaften dominieren

### Dispersionen

Das gegebenenfalls teilneutralisierte (Meth)acrylat-Copolymer kann z. B. in Form einer wäßrigen Dispersion mit 10 bis 50-prozentigen Feststoffanteil vorliegen.

Das gegebenenfalls teilneutralisierte (Meth)acrylat-Copolymer kann in Form eines redispergierbaren Pulvers vorliegen, welches aus einer Dispersion z. B. durch Sprühtrocknung gewonnen wurde.

### Dispersionen / Teilneutralisation

Das Emulsionspolymerisat wird vorzugsweise in Form einer 10- bis 50-gew.-prozentigen, insbesondere 20 bis 40-prozentigen wäßrigen Dispersion erzeugt und angewendet. Als Handelsform ist ein Feststoffgehalt von 30 Gew.-% bevorzugt. Für die Verarbeitung ist eine teilweise Neutralisation der Methacrylsäure-Einheiten entbehrlich; sie ist jedoch, beispielsweise in einem Umfang bis zu 5 oder 10 Mol-% möglich, wenn eine Stabilisierung oder Verdickung der Überzugsmitteldispersion erwünscht sein sollte. Der Gewichtsmittelwert Latex-Teilchengröße (Radius) beträgt in der Regel 40 bis 100 nm, vorzugsweise 50 bis 70 nm, was eine verarbeitungstechnisch günstige Viskosität unter 1000 mPa · s gewährleistet. Die Teilchengröße kann durch Laserbeugung, z. B. mit dem Mastersizer 2000 (Fa. Malvern), bestimmt werden.

Bei höheren Neutralisationsgrades z. B. 10 bis 50 Mol.-% oder vollständiger Neutralisation ist es möglich, das Copolymer in einen gelösten Zustand zu überführen.

Um eine Lösung des anionischen Copolymers herzustellen ist in der Regel eine teilweise oder vollständige Neutralisation der Säuregruppen nötig. Das anionische Copolymer kann z. B. nach und nach in einer Endkonzentration von 1 bis 40 Gew.-% in Wasser eingerührt werden und dabei teilweise oder vollständig neutralisiert werden durch Zugabe einer basischen Substanz wie z. Lysin oder Arginin. Es ist auch möglich ein Pulver des Copolymeren einzusetzen, dem bereits bei seiner Herstellung zum Zweck der (Teil)neutralisation eine Base z. B. Lysin zugesetzt wurde, so dass das Pulver ein bereits (teil)neutralisiertes Polymer ist. Der pH-Wert der Lösung liegt in der Regel über 4, z. B. im Bereich von 4 bis ca. 7. Man kann dabei auch z. B. Mischungen von Batches von voll- oder teilneutralisierten Dispersionen mit nicht neutralisierten Dispersionen vornehmen und in beschriebener Weise weiterverarbeiten, d. h. die Mischung für Überzüge verwenden oder zunächst zu einem Pulver gefrier- oder sprühtrocknen.

Die Dispersion kann z. B. auch in an sich bekannter Weise sprühgetrocknet oder gefriergetrocknet werden und im Form eines redispergierbaren Pulvers bereitgestellt werden (siehe z. B. EP-A 0 262 326). Alternative Verfahren sind die Gefriertrocknung oder Coagulation uns Abquetschen des Wassers in einem Extruder mit anschließender Granulation (siehe z. B. EP-A 0 683 028).

Copolymer-Dispersionen aus sprüh- oder gefriergetrockneten und redispergierten Pulvern können eine erhöhte Scherstabilität aufweisen. Dies ist insbesondere beim Sprühauftrag von Vorteil. Dieser Vorteil tritt insbesondere verstärkt hervor wenn das in der Dispersion enthaltene Copolymer zu 2 bis 10, bevorzugt zu 5 bis 7 Mol-% in teilneutralisierter form vorliegt (bezogen auf die im Copolymer enthaltenen Säuregruppen). Bevorzugt ist zu diesem Zweck die Teilneutalisation mittels Zugabe von Lysin oder Arginin. Bevorzugt ist ein anionischer Emulgator in Menge von 0,1 bis 2 Gew.-% enthalten. Besonders bevorzugt ist Natiumlaurylsulfat als Emulgator.

### Verwendung des teilneutralisierten (Meth)acrylatcopolymeren

Das mit einer kationischen, organischen Base mit einem M_{w} > 150 oder mit Lysin teilneutralisierte, anionische (Meth)acrylat-Copolymere kann bevorzugt als Überzugsmittel für die erfindungsgemäße Arzneiform verwendet werden. Dabei werden in etwa 90 %, bevorzugt 95 oder 100 % des enthaltenen Wirkstoffs im Freigabetest nach USP 28 für 2 Stunden bei pH 1,2 und anschließendem Umpuffern auf den pH-Wert des Beginns der Wirkstofffreisetzung nach dem Umpuffern in höchstens 90 %, bevorzugt höchstens 75 %, insbesondere höchstens 50 % der Zeit frei, die bei einer vergleichbaren Arzneiform mit gleichem Polymerzug, jedoch ohne oder Teilneutralisation mittels anderer Basen, dafür verstreicht.

Setzt eine nicht mit Lysin oder mit einer kationischen, organischen Base mit einem M_{w} > 150 teilneutralisierte Arzneiform den Wirkstoff im Freigabetest nach USP 28 für 2 Stunden bei pH 1,2 und anschließendem Umpuffern auf den pH-Wert des Beginns der Wirkstofffreisetzung, z.B. pH 5,5, in z. B. 120 min nach dem Umpuffern zu 90 % frei, benötigt eine vergleichbare Arzneiform, deren Überzug mittels Lysin oder einer kationischen, organische Basen mit einem M_{w} > 150 teilneutralisiert wurde, dafür höchstens 108 min (90 % der Zeit), höchstens 90 min (75 %) oder höchstens 60 min (50 %).

Dem Fachmann ist der angegebene Freisetzungstest nach USP 28, insbesondere nach USP 28 <711> Paddle-Methode (= Apparatus 2), hinlänglich bekannt

Der typische Testablauf ist der Folgende:
1. Die Gefäße der Freisetzungsaparatur werden mit je 360mL 0,1M-HCl (pH 1,2) gefüllt und die Temperatur des Wasserbads auf 37 ± 0,5°C eingestellt.
2. Der Blattrührer wird mit einer Umdrehungsrate von 100rpm angeschaltet.
3. In jedes Gefäß der Apparatur werden 1 g Pellets gegeben. Es wird darauf geachtet, dass keine Luftblasen auf der Pellet-Oberfläche sind.
4. Nach 120 min werden 140mL Phosphat-Pufferlösung (auf 37°C temperiert) zugesetzt, so dass im Endvolumen von 500mL der gewünschte pH-Wert entsteht: pH 5,5; 5,6; 5,7; 5,8 oder 7,0.
5. Bestimmung des Zeitpunktes der 100%-igen Wirkstofffreigabe, je nach Wirkstoff, z. B. im Falle von Theophyllin photometrisch bei 271 nm, im Umlaufverfahren.

### Arzneiform

Die Erfindung betrifft eine Arzneiform, enthaltend einen wirkstoffhaltigen Kern, der mit einer Überzugsschicht aus einem magensaftresistenten, darmsaftlöslichen (Meth)acrylat-Copolymer umhüllt ist, wobei sich zwischen dem Kern und der Überzugsschicht eine Trennschicht, enthaltend ein filmbildendes wasserlösliches Polymer, befindet, dadurch gekennzeichnet, dass die Trennschicht dreischichtig ausgeführt ist, wobei eine innere Schicht mit einer wasserabweisenden Substanz vorhanden ist und zwei Schichten des filmbildenden wasserlöslichen Polymeren die Schicht mit der wasserabweisenden Substanz einschließen.

Die Trennschicht ist dreischichtig ausgeführt, wobei zwei Schichten des filmbildenden wasserlöslichen Polymeren eine Schicht mit der wasserabweisenden Substanz einschließen.

Die Arzneiform kann bevorzugt einen Polymerüberzug mit Lysin oder Arginin als Teilneutralisationsmittel in Kombination mit 5 bis 25 Gew.-% eines Weichmachers, bezogen auf das Polymer, enthalten.

Die erfindungsgemäße Arzneiform kann z. B. in Form einer multipartikulären Arzneiform, pellethaltigen Tabletten, Minitabletten, Kapseln, Sachets, Brausetabletten oder Trockensäften vorliegen.

### Verfahren zur Herstellung einer Arzneiform

Die Erfindung betrifft weiterhin ein Verfahren zur Herstellung der erfindungsgemäßen Arzneiform in an sich bekannter Weise mittels pharmazeutisch üblicher Verfahren, wie direktem Verpressen, Verpressen von Trocken-, Feucht- oder Sintergranulaten, Extrusion und anschließende Ausrundung, feuchte oder trockene Granulation oder direkte Pelletierung oder durch Binden von Pulvern (Powder layering), durch Aufsprühen von Suspensionen oder Lösungen auf wirkstofffreie Kugeln bzw. neutrale Kerne (Nonpareilles) oder wirkstoffhaltige Partikeln und mittels Auftrag des Polymerüberzugs im Sprühverfahren oder durch Wirbelschichtgranulation.

### Herstellung multipartikulärer Arzneiformen

Die Erfindung eignet sich insbesondere zur Herstellung multipartikulärer Arzneiformen, da das umhüllende (Meth)acrylat-Copolymer den hohen Drücken beim Verpressen der Pellets mit dem Füllstoff standhält.

Die Herstellung von multipartikulären Arzneiformen durch Verpressen eines pharmazeutisch üblichen Bindemittels mit wirkstoffhaltigen Partikeln ist z. B. Beckert et al. (1996), "Compression of enteric-coated pellets to disintegrating tabletts", International Journal of Pharmaceutics 143, S. 13 - 23, und in WO 96/01624 ausführlich beschrieben.

Wirkstoffhaltige Pellets können hergestellt werden indem man mittels eines Layeringprozesses Wirkstoff aufbringt. Dazu wird Wirkstoff gemeinsam mit weiteren Hilfsstoffen (Trennmittel, ggf. Weichmacher) homogenisiert und in einem Bindemittel gelöst oder suspendiert. Mittels eines Wirbelschichtverfahrens kann die Flüssigkeit auf Placebopellets oder sonstige geeignete Trägermaterialien aufgebracht werden, wobei das Lösungs- oder Suspendiermittel verdunstet wird (Literatur: International Journal of Pharmaceutics 143, S. 13 - 23*)*. Nach dem Herstellverfahren kann sich ein Trocknungsschritt anschließen. Der Wirkstoff kann in mehreren Schichten aufgebracht werden.

Einige Wirkstoffe, z. B. Acetylsalicylsäure, sind in Form von Wirkstoffkristallen handelsüblich und können in dieser Form anstelle von wirkstoffhaltigen Pellets eingesetzt werden.

Filmüberzüge auf wirkstoffhaltige Pellets werden üblicherweise in Wirbelschichtgeräten aufgebracht. Rezepturbeispiele sind in dieser Anmeldung erwähnt. Filmbildner werden üblicherweise mit.Weichmachern und Trennmitteln nach einem geeigneten Verfahren gemischt. Hierbei können die Filmbildner als Lösung oder Suspension vorliegen. Die Hilfsstoffe für die Filmbildung können ebenfalls gelöst oder suspendiert sein. Organische oder wässrige Löse- oder Dispergiermittel können verwendet werden. Zur Stabilisierung der Dispersion können zusätzlich Stabilisatoren verwendet werden (Beispiel: Tween 80 oder andere geeignete Emulgatoren bzw. Stabilisatoren).

Beispiele für Trennmittel sind Glycerolmonostearat oder andere geeignete Fettsäurederivate, Kieselsäurederivate oder Talkum. Beispiele für Weichmacher sind Propylenglykol, Phthalate, Polyethylenglykole, Sebacate oder Citrate, sowie andere in der Literatur erwähnte Substanzen.

Zwischen wirkstoffhaltiger und darmlöslicher Copolymer-Schicht kann eine trennende Schicht aufgebracht sein, die der Trennung von Wirkstoff und Überzugsmaterial zum Zwecke der Verhinderung von Interaktionen dient. Diese Schicht kann aus inerten Filmbildnern (z.B. HPMC, HPC oder (Meth)acrylsäure-Copolymeren) oder z.B. Talkum oder anderen geeigneten pharmazeutischen Substanzen bestehen. Ebenso können Kombinationen aus Filmbildnern und Talkum oder ähnlichen Stoffen verwendet werden.

Es ist auch möglich eine Trennschicht aus teilweise bzw. vollneutralisierten Copolymer-Dispersionen aufzubringen.

Mischungen zur Herstellung von Tabletten aus überzogenen Partikeln werden durch Vermischen der Pellets mit geeigneten Bindemitteln für die Tablettierung, nötigenfalls der Zugabe von zerfallsfördernden Substanzen und nötigenfalls der Zugabe von Schmiermitteln zubereitet. Das Mischen kann in geeigneten Maschinen stattfinden. Ungeeignet sind Mischer, die zu Schäden an den überzogenen Partikeln führen, z. B. Pflugscharmischer. Zur Erzielung geeigneter kurzer Zerfallszeiten kann eine spezielle Reihenfolge bei der Zugabe der Hilfsstoffe zu den überzogenen Partikeln erforderlich sein. Durch Vormischung mit der überzogenen Partikel mit dem Schmier- oder Formentrennmittel Magnesiumstearat kann dessen Oberfläche hydrophobisiert und somit Verkleben vermieden werden.

Zum Tablettieren geeignete Mischungen enthalten üblicherweise 3 bis 15 Gew.-% eines Zerfallshilfsmittels, z. B. Kollidon CL und z. B. 0,1 bis 1 Gew.-% eines Schmier- und Formentrennmittels wie Magnesiumstearat. Der Bindemittelanteil bestimmt sich nach dem geforderten Anteil an überzogenen Partikeln.

Typische Bindemittel sind z. B. Cellactose^{®}, mikrokristalline Cellulose, Calciumphosphate, Ludipress^{®}, Lactose oder andere geeignete Zucker, Calciumsulfate oder Stärkederivate. Bevorzugt werden Substanzen mit geringer Schüttdichte.

Typische Zerfallshilfsmittel (Sprengmittel) sind quervernetzte Stärke- oder Cellulosederivate, sowie quervernetztes Polyvinylpyrrolidon. Ebenso sind Cellulosederivate geeignet. Durch Auswahl eines geeigneten Bindemittels kann die Verwendung von Zerfallshilfsmittel entfallen.

Typische Schmier- und Formentrennmittel sind Magnesiumstearate oder andere geeignete Salze von Fettsäuren oder in der Literatur zu diesem Zweck aufgeführte Substanzen (z.B. Laurinsäure, Calciumstearat, Talkum usw.). Bei Verwendung geeigneter Maschinen (z.B. Tablettenpresse mit externer Schmierung) oder geeigneter Formulierungen kann die Verwendung eines Schmier- und Formentrennmittels in der Mischung entfallen.

Der Mischung kann gegebenenfalls ein Hilfsmittel zur Fließverbesserung beigefügt sein (z. B. hochdisperse Kieselsäurederivate, Talkum usw.).

Das Tablettieren kann auf üblichen Tablettenpressen, Exzenter- oder Rundlauftablettenpressen erfolgen, bei Preßkräften im Bereich von 5 bis 40 kN, bevorzugt 10 - 20 kN. Die Tablettenpressen können mit Systemen zur externen Schmierung ausgestattet sein. Gegebenenfalls kommen spezielle Systeme zur Matrizenbefüllung zum Einsatz, die die Matrizenbefüllung mittels Rührflügeln vermeiden.

### Weitere Herstellungsverfahren für die erfindungsgemäße Arzneiform

Auftragsverfahren erfolgt mittels Sprühauftrag aus organischer Lösung, oder bevorzugt wässrigen Dispersionen durch Schmelzen oder durch direkten Pulverauftrag. Für die Ausführung ist dabei entscheidend, dass gleichmäßige, porenfreie Überzüge entstehen.

Auftragsverfahren gemäß Stand der Technik s. z.B. Bauer, Lehmann, Osterwald, Rothgang, "Überzogene Arzneiformen" Wissenschaftliche Verlagsgesellschaft mbH Stuttgart, Kap 7, S.165 - 196

Für die Applikation sind relevante Eigenschaften, geforderte Tests und Spezifikationen in Arzneibüchern aufgelistet.

Details sind den gängigen Lehrbüchern zu entnehmen, z.B.:
- Voigt, R. (1984): Lehrbuch der pharmazeutischen Technologie; Verlag Chemie Weinheim - Beerfield Beach/Florida - Basel.
- Sucker, H., Fuchs, P., Speiser, P. : Pharmazeutische Technologie, Georg Thieme Verlag Stuttgart (1991), insbesondere Kapitel 15 und 16, S. 626 - 642.
- Gennaro, A.,R. (Editor), Remington's Pharmaceutical Sciences, Mack Publishing Co., Easton Pennsylvania (1985), Chapter 88, S. 1567 - 1573.
- List, P. H. (1982): Arzneiformenlehre, Wissenschaftliche Verlagsgesellschaft mbH, Stuttgart.

### Hilfsstoffe

Den erfindungsgemäßen Formulierungen können bei der Herstellung übliche Hilfs- bzw. Zuschlagstoffe hinzugefügt werden. Grundsätzlich müssen natürlich alle eingesetzten Substanzen toxikologisch unbedenklich und insbesondere in Arzneimitteln ohne Risiko für Patienten zu verwenden sein.

Einsatzmengen und Verwendung der üblichen Zuschlagstoffe in Arzneimittelüberzügen oder Beschichtungen sind dem Fachmann geläufig. Übliche Zuschlagstoffe können z. B. Weichmacher, Trennmittel, Pigmente, Stabilisatoren, Antioxidantien, Porenbildner, Penetrationsförderer, Glanzmittel, Aromastoffe, Detergenzien, Schmierstoffe oder Geschmacksmittel sein. Sie dienen als Verarbeitungshilfsmittel und sollen ein sicheres und reproduzierbares Herstellungsverfahren sowie gute Langzeitlagerstabilität gewährleisten oder sie erreichen in der Arzneiform zusätzliche vorteilhafte Eigenschaften. Sie werden den Polymerzubereitungen vor der Verarbeitung zugesetzt und können die Permeabilität der Überzüge beeinflussen, was ggf. als zusätzlicher *Steuerparameter genutzt werden kann.*

### • Trennmittel:

Trennmittel besitzen in der Regel lipophile Eigenschaften und werden in der Regel den Sprühsuspensionen zugesetzt. Sie verhindern eine Agglomeration der Kerne während der Befilmung. Bevorzugt werden Talkum, Mg- oder Ca - Stearat, gemahlene Kieselsäure, Kaolin oder nicht ionische Emulgatoren mit einem HLB - Wert zwischen 3 und 8 eingesetzt. Übliche Einsatzmengen für Trennmittel in den erfindungsgemäßen Überzugs- und Bindemitteln liegen zwischen 0,5 bis 100 Gew.-% bezogen auf das Copolymer.

### • Pigmente:

Mit dem Überzugsmittel unverträgliche Pigmente sind insbesondere solche Pigmente, die wenn sie der (Meth)acrylat-Copolymer-Dispersion direkt zugesetzt werden, z. B. durch Einrühren, in üblichen Anwendungsmengen von z. B. 20 bis 400 Gew.-% bezogen auf das Trockengewicht des (Meth)acrylat-Copolymeren zur Destabilisierung der Dispersion, Koagulation, zu Entmischungserscheinungen oder ähnlich unerwünschten Effekten führen. Weiterhin sind die zu verwendenden Pigmente natürlich nicht toxisch und für pharmazeutische Zwecke geeignet. Siehe dazu z. B. auch: Deutsche Forschungsgemeinschaft, Farbstoffe für Lebensmittel, Harald Boldt Verlag KG, Boppard (1978); Deutsche Lebensmittelrundschau 74, Nr. 4, S. 156 (1978); Arzneimittelfarbstoffverordnung AmFarbV vom 25.08.1980.

Mit dem Überzugsmittel unverträgliche Pigmente können z. B. Aluminiumoxidpigmente sein. Unverträgliche Pigmente sind z. B., Gelborange , Cochenillerotlack, Farbpigmente auf Basis von Aluminiumoxid bzw Azofarbstoffen, Sulfonsäurefarbstoffe, Gelborange S (E110, C.I. 15985, FD&C Yellow 6), Indigocarmin (E132, C.I. 73015, FD&C Blue 2), Tartrazin (E 102, C.I. 19140, FD&C Yellow 5), Ponceau 4R (E 125, C.I. 16255, FD&C Cochineal Red A), Chinolingleb (E 104, C.I. 47005, FD&C Yellow 10), Erythrosin (E127, C.I. 45430, FD&C Red 3), Azorubin (E 122, C.I. 14720, FD&C Carmoisine), Amaranth (E 123, C. I. 16185, FD&C Red 2), Brilliantsäuregrün (E 142, C.I. 44090, FD&C Green S).

Die angegebenen E-Nummern der Pigmente beziehen sich auf eine EU-Nummerierung. Siehe dazu auch "Deutsche Forschungsgemeinschaft, Farbstoffe für Lebensmittel, Harald Boldt Verlag KG, Boppard (1978); Deutsche Lebensmittelrundschau 74, Nr. 4, S. 156 (1978); Arzneimittelfarbstoffverordnung AmFarbV vom 25.08.1980. Die FD&C-Nummern beziehen sich auf die Zulassung in Food, Drugs und Cosmetics durch U.S. Food and Drug Administration (FDA) beschrieben in: U.S. Food and Drug Administration, Center for Food Safety and Applied Nutrition, Office of Cosmetics and Colors: Code of Federal Regulations - Title 21 Color Additive Regulations Part 82, Listing of Certified Provisionally Listed Colors and Specifications (CFR 21 Part 82).

### • Weichmacher

Weitere Zuschlagstoffe können auch Weichmacher sein. Übliche Mengen liegen zwischen 0 und 50, bevorzugt 2 bis 20, insbesondere 5 bis 10 Gew.-%.

Weichmacher können je nach Typ (lipophil oder hydrophil) und zugesetzter Menge die Funktionalität der Polymerschicht beeinflussen. Weichmacher erreichen durch physikalische Wechselwirkung mit dem Polymeren eine Absenkung der Glasübergangstemperatur und fördern in Abhängigkeit von der zugesetzten Menge die Verfilmung. Geeignete Stoffe haben in der Regel ein Molekulargewicht zwischen 100 und 20.000 und enthalten eine oder mehrere hydrophile Gruppen im Molekül, z. B. Hydroxyl-, Ester- oder Aminogruppen.

Beispiele geeigneter Weichmacher sind Citronensäurealkylester, Glycerinester, Phthalsäurealkylester, Sebacinsäurealkylester, Succroseester, Sorbitanester, Diethylsebacat, Dibutylsebacat und Polyethylenglykole 200 bis 12.000. Bevorzugte Weichmacher sind Triethylcitrat (TEC) und Acetyltriethylcitrat (ATEC). Weiterhin zu nennen sind in der Regel bei Raumtemperatur flüssige Ester wie Citrate, Phthalate, Sebacate oder Rizinusöl. Bevorzugt werden Zitronensäure- und Sebacinsäureester verwendet.

Die Zugabe der Weichmacher zur Formulierung kann in bekannter Weise, direkt, in wäßriger Lösung oder nach thermische Vorbehandlung der Mischung vorgenommen werden. Auch können Mischungen von Weichmachern eingesetzt werden

### Emulgatoren

Emulgatoren werden in der Regel eingesetzt, um die Sprühfähigkeit und die Flexibilität der resultierenden Schichten oder Filme zu verbessern. Der Einsatz von Emulgatoren kann z. B. in einer Konzentration von 0,1 bis 50 Gew.-% bezogen auf Gesamtgewicht der betroffenen Schicht oder des Films betragen.

Für Schichten aus wasserlöslichen Polymeren, wie z. B. der oder den Schichten der Trennschicht mit dem filmbildenden wasserlöslichen Polymer, können Emulgatoren mit einem HLB-Wert nach Griffin von größer 8 eingesetzt werden. Bevorzugt sind z. B. Natriumdodecylsulfat, Polysorbate (Tween® 20 bis 80), Polyoxyethylen-Polyoxypropylen-Blockpolymerisate (Poloxamer®, Pluronic®), Polyethylenglykol-Fettalkohol-Ether (Cremophor®), Polyoxyethylenfettalkoholether (Brij®), Saccharosefettsäureester (Crodesta®). Polyoxyethylen-Stearylalkohole (Emulgin®, Cetomacrogol®).

Für Schichten aus wasserabweisenden Substanzen, wie z. B. der Schicht der Trennschicht mit der wasserabweisenden Substanz, können Emulgatoren mit einem HLB-Wert nach Griffin 8 oder kleiner 8 eingesetzt werden. Dadurch kann insbesondere die micellare Auflösung der wasserabweisenden Schicht nochmals beschleunigt werden. Der Emulgator-Zusatz kann somit als weiteres Steuerungselement verwendet werden.

Beispiele für Emulgatoren mit HLB-Werten von 8 oder kleiner 8 sind: Wollwachsalkohole (Agnowax®, Hartolan®, Eucerit®), Glycerinester von Fettsäuren, wie z. B. Glycerinmonooleat, Glycerinmonococoat oder Glycerinmonolaurat, Sorbitanester, wie z. B. Sorbitan-Trioleat (Span® 85), Sorbitan-Monostearat, (Span® 60. Aclacel® 80), Sorbitan- Monopalmitat (Crill® 1. Arlacel® 20, Span® 20), Saccharose-Ester, wie z. B. Saccharose-Tristearat, Saccharose-Distearat, Saccharose-Dipalmitat. PEG (200)-monostearat oder Ricinusöl hydriert

Der HLB-Wert ist ein 1950 von Griffin eingeführtes Maß der Hydrophilie bzw. Lipophilie von nichtionischen Tensiden. Er lässt sich experimentell durch die Phenol-Titrationsmethode nach Marszall bestimmen; vgl. "Parfümerie, Kosmetik", Band 60, 1979, S. 444 - 448; weitere Literaturhinweise in Römpp, Chemie-Lexikon, 8.Aufl. 1983, S.1750. Siehe weiterhin z. B. US 4 795 643 (Seth)).

Ein HLB-Wert (Hydrophile/Lipophile Balance) lässt sich nur bei nicht ionischen Emulgatoren exakt bestimmen. Bei anionischen Emulgatoren kann dieser Wert rechnerisch ermittelt werden, liegt jedoch praktisch immer über oder weit über 14.

### Wirkstoffe/Arzneistoffe

Gebräuchliche Arzneistoffe sind in Nachschlagewerken, wie z.B. der Roten Liste oder dem Merck Index zu entnehmen.

Die im Sinne der Erfindung eingesetzten Arzneistoffe sind dazu bestimmt, am oder im menschlichen oder tierischen Körper Anwendung zu finden, um
1. Krankheiten, Leiden, Körperschäden oder krankhafte Beschwerden zu heilen, zu lindern, zu verhüten oder zu erkennen.
2. die Beschaffenheit, den Zustand oder die Funktionen des Körpers oder seelische Zustände erkennen lassen.
3. vom menschlichen oder tierischen Körper erzeugte Wirkstoffe oder Körperflüssigkeiten zu ersetzen.
4. Krankheitserreger, Parasiten oder körperfremde Stoffe abzuwehren, zu beseitigen oder unschädlich zu machen oder
5. die Beschaffenheit, den Zustand oder die Funktionen des Körpers oder seelische Zustände zu beeinflussen.

### Therapieklassen

Diese pharmazeutisch aktiven Substanzen können einer oder mehrerer Wirkstoffklassen angehören, wie ACE-Hemmer, Adrenergika, Adrenocortikosteroide, Aknetherapeutika, Aldose-Reduktase-Hemmer, Aldosteron-Antagomsten Alpha-Glucosidasehemmer, Alpha 1-Antagonisten, Mittel gegen Alkoholabusus, Aminosäuren, Amöbizide, Anabolika, Analeptika, Anaesthetika-Zusätze. Anaesthetika (nicht inhalativ), Anaesthetika (lokal), Analgetika. Androgene, Anginatherapeutika, Antagonisten, Antiallergika Antiallergika wie PDE-Hemmer, Antiallergika zur Asthmabehandlung, Weitere Antiallergika (z.B Leukotrienantagonisten) Antianämika, Antiandrogene, Antianxiolytika Antiarthritika, Antiarrhythmika, Antiatheriosklerotika, Antibiotika. Anticholinergika, Anticonvulsiva, Antidepressiva, Antidiabetika, Antidiarrhoika, Antidiuretika, Antidots, Antiemetika, Antiepileptika. Antifibrinolytika, Antiepileptika, Antihelmintika, Antihistaminika, Antihypotensiva, Antihypertensiva, Antihypertonika, Antihypotonika, Antikoagulantien, Antimykotika, Antiöstrogene, Antiöstrogene (Nicht-Steroide), Antiparkinson-Mittel, Antiphlogistika, antiproliferative Wirkstoffe, antiprotozoen Wirkstoffe, Antirheumatika, Antischistosomizide, Antispasmolytika, Antithromboika, Antitussiva Appetitzügler, Arteriosklerosemittel, Bakteriostatika, Betarezeptorenblocker, Bronchodilatoren, Carboanhydrase-Hemmer, Chemotherapeutika, Choleretika, Cholinergika, Cholinesterase-Hemmer, Mittel zur Behandlung von Colitis ulcerosa. Cyclooxigenasehemmer, Diuretika Ektoparasitizide, Emetika, Enzyme, Enzyminhibitoren, Fibrinolytika, Fungistatika, Gichtmittel Glaukomtherapeutika, Glucocorticoide, Glucocortikosteroide Hamostatika, Herzglykoside, Histamin H2-Antagonisten, Hormone und deren Hemmstoffe, Immuntherapeutika, Kardiotonika, Kokkidiostatika, Laxantien, Lipidsenker, Magen-Darmtherapeutika, Malariatherapeutika, Migränemittel, Mikrobiozide, Mittel zur Behandlung von Morbus Crohn, Metastasenhemmer, Migränemittel, Mineralstoffpräparate, motilitätssteigernde Wirkstoffe, Muskelrelaxantien, Neuroleptika, Wirkstoffe zur Behandlung der Osteoporose, Otologika, Parkinsonmittel, Phytopharmaka, Protonenpumpenhemmer, Prostaglandine, Wirkstoffe zur Behandlung der benignen Prostatahyperblasie, Wirkstoffe zur Behandlung des Pruritus, Psoriasis Wirkstoffe Psychopharmaka, Radikalfänger, Renin-Antagonisten, Schilddrüsentherapeutika, Wirkstoffe zur Behandlung von Seborrhoe, Spasmolytika, alpha- und beta-Sympatomimetika, Tenatoprazol, Thrombozytenaggregationshemmer, Tyrosinkinaseinhibitoren, Tranquilizer, Ulkustherapeutika, Mittel zur Behandlung der Urolithiasis, Virustatika, Vitamine, Zytokine, Zytostatika.

### Wirkstoffe

Beispiele geeigneter Wirkstoffe sind Acarbose Acetylsalicylsäure, Abacavir, Aceclofenac Aclarubicin, Acyclovir, Actinomycin, Adalimumab, Adefovir, Adefovirdipivoxil, Adenosylmethionin, Adrenalin und Adrenalinderivate, Agalsidase alpha Agalsidase beta, Alemtuzumab, Alfuzosin, Almotriptan, Allopurinol, Almotriptan, Alosetron, Alphacept, Alprazolam, Alprostadil, Amantadin, Ambroxol, Amisulprid, Amlodipin, Amoxicillin, 5-Aminosalicylsäure, Amitriptylin, Amlodipin, Amoxicillin, Amprenavir, Anagrelid, Anakinra, Anastrozol, Androgen und Androgenderivate, Apomorphin, Aripiprazol, Arsentrioxid, Artemether, Atenolol, Atorvastatin, Atosiban, Azathioprin, Azelainsäure, Barbitursäurederivate, Balsalazid, Basiliximab, Beclapermin, Beclomethason, Bemiparin, Benazepril, Benzodiazepine, Beraprost, Betahistin, Bexaroten Bezafibrat, Bicalutamid. Bimatoprost Bismuth citrate. Bismuth subsalicylate, Bosentan, Botulinumtoxim, Brimonidin, Brinzolamid, Bromacepam, Bromocriptin, Budesonid, Budipin, Bufexamac, Bumetanid, Buprenorphin, Bupropion, Butizin, Calcitonin, Calciumantagonisten, Calciumcarbonat, Calciumdobesilat, Calciumsalze, Camazepam, Candesartan, Capecitabin, Captopril, Carbamazepin, Carifenacin, Carvedilol, Caspofungin, Cefaclor, Cefadroxil, Cefalexin Cefalosporine, Cefditoren, Cefprozil, Cefuroxim, Celecoxib, Cepecitabin, Cerivastatim, Cetirizin, Cetrorelix, Cetuximab, Chenodeoxycholsäure, Chlordiazepoxid. Choriogonadotropin, Ciclosporin, Cidofovir, Cilazapril, Cimetidin, Ciprofloxacin, Cisplatin, Cladribin, Clarithromycin, Clavulansäure, Clindamycin, Clobazam, Clobutinol, Clonazepam, Clonidin, Clopidogrel, Codein, Coffein, Colestyramin, Cromoglicinsaure, Cotrimoxazol, Cumarin und Cumarinderivate, Cysteamin, Cystein, Cytarabin, Cyclophosphamid, Cyproteron Cytarabin, Daclizumab, Dalfopristin, Danaparoid, Dapiprazol, Darbepoetin, Defepripron, Desferrixamin, Desipramin, Desirudin, Desloaratadin, Desmopressin, Desogestrel, Desonid, Dexibuprofen, Dexketoprofen, Didanosin, Disoproxil, Diazepam und Diazepamderivate, Didanosin, Dihydralazin, Diltiazem, Dimenhydrinat, Dimethylsulfoxid, Dimeticon, Dipivoxil, Dipyridarnoi, Dolasetron, Domperidon und Domperidanderivate, Donepzil, Dopamin, Doxazosin, Doxorubizin, Doxylamin, Diclofenac, Divalproex, Dronabinol, Drospirenon, Drotrecogin alpha, Duloxetin, Dutasterid, Ebastin, Econazol, Efavirenz, Eletripan, Emidastin, Emtricitabin, Enalapril, Encepur, Entacapon, Enfurvirtid, Ephedrin, Epinephrin, Eplerenon, Epoetin und Epoetinderivate, Eprosartan, Eptifibatid, Ertapenem, Esomeprazol, Estrogen und Estrogenderivate, Etanercept, Ethenzamid, Ethinöstradiol, Etofenamat, Etofibrat, Etofyllin, Etonogestrel, Etoposid, Etoricoxib, Exemestan, Ezetimib, Famciclovir, Famotidin, Faropenandaloxat, Felodipin, Fenofibrat, Fenofibrinsäure, Fenoldopam, Fentanyl, Fenticonazol, Fexofenadin, Finasterid, Fluconazol, Fludarabin, Flunarizin, Fluorouracil, Fluoxetin, Flurazepam. Flurbiprofen, Flupirtin, Flutamid, fluvastatin, Follitropin, Fomivirsen, Fondaparinux, Formoterol, Fosfomicin, Fosinopril, Frovatriptan, Furosemid, Fusidinsaure, Gabapentin, Gadobenat, Galantamin, Gallopamil, Ganciclovir, Ganirelix, Gatifloxacin, Gefitinib, Gemfibrozil, Gemopatrilat, Gentamicin, Gepiron, Gestagen und Gestagenderivate, Ginkgo, Glatiramer, Glibenclamid, Glimepride, Glipizide, Glucagon, Glucitol und Glucitolclerivate, Glucosamin und Glucosaminderivate, Glykosidantibiotika, , Glutathion, Glycerol und Glycerolderivate, Hypothalamushormone, Goserelin, Granisetron, Grepafloxacin, Gyrasehemmer, Guanethidin, Gyrasehemmer, Hämin, Halofantrin, Haloperidol, Harnstoffderivate als orale Antidiabetika, Heparin und Heparinderivate, Herzglykoside, Hyaluronsäure, Hydralazin, Hydrochlorothiazid und Hydrochlorothiazidderivate, Hydroxyomeprazol, Hydroxyzin. Ibritumomab, Ibuprofen, Idarubicin, Ifliximab, Ifosfamid, Iloprost, Imatinib, Imidapril, Imiglucerase, Imipramin, Imiquimod, Imidapril, Indometacin, Indoramin, Infliximab, Insulin, Insulin glargin, Interferone, Irbesartan, Irinotecan, Isoconazol, Isoprenalin, Isorbid-Mononitrat, Isorbid-Dinitrat, Itraconazol, Ivabradine, Jod und Jodderivate, Johanniskraut, Kaliumsalze, Ketoconazol, Ketoprofen. Ketotifen, Lacidipin, Lamivudin, Lamotrigin, Lansoprazol, Laronidase, Latanoprost, Leflunomid, Leminoprazol Lepirudin, Lercanidipin, Letepnnim, Letrozol, Levacetytmethadol, Levetiracetam, Levocetirizin, Levodopa, Levodrpropicin, Levofloxacin, Levomethadon, Licofelone, Linezolid, Lipinavir, Liponsäure und Liponsaurederivate, Lisinopril, Lisurid, Lofepramin, Lodoxamid, Lomefloxacin, Lomustin, Loperamid, Lopinavir, Loratadin, Lornoxicam, Losartan, Lovastatin, Lumefantnn Lutropine Magnesiumsalze, Makrolidantibiotika, Mangafodipir, Maprotilin, Mebendazol, Mebeverin Meclozin, Mefenaminsaure. Mefloquin Meloxicam, Memantin, Mepindolol, Meprobamat, Meropenem, Mesalazin, Mesoprostol, Mesuximid, Metamizol, Metaxalon, Metformin, Methadon, Methotrexat, Methyl-(5-amino-4-oxopentanoat), Methylnaloxon, Methylnaltrexone, Methylphenidat Methylprednisolon, Metixen, Metoclopramid, Metoprolol, Metronidazol, Mianserin. Mibefradil, Miconazol, Mifepriston, Miglitol, Miglustad, Milnacipran, Minocyclin, Minoxidil, Misoprostol, Mitomycin, Mizolastin, Modafinil, Moexipril, Molsidomin, Montelukast, Moroctocog, Morphinane, Morphin und Morphinderivate, Moxifloxacin, Mutterkornalkaloide, Nalbuphin, Naloxon, Naproxen. Naratriptan, Narcotin, Natamycin, Nateglinid, Nebivolol. Nefazodon, Nelfinavir, Neostigmin, Neramexan, Nevirapin, Nicergolin, Nicethamid, Nifedipin, Nifluminsäure, Nilutamid, Nimodipin, Nimorazol, Nimustin, Nesintid, Nisoldipin, Nizatidin, Norfloxacin, Novaminsulfon, Noscapm, Nystatin, Ofloxacin, Oktotride, Olanzapin, Olmesartan, Olsalazin, Oseltamivir, Omapatrilat, Omeprazol, Omoconazol, Ondansetron, Orlistat, Oseltamivir, Oxaceprol, Oxacillin, Oxaliplatin, Oxaprozin, Oxcarbacepin, Oxibutin, Oxicodon, Oxiconazol, Oxybutymin Oxycodone, Oxymetazolin, Palivizumab, Palonosetron, Pantoprazol, Paracetamol, Parecoxib, Paroxetin, Pegaspargase, Peg-Interferon, Pegfilgrastrim, Penciclovir, orale Penicilline, Pentazocin, Pentifyllin, Pentoxifyllin, Peptidantibiotika, Perindopril, Perphenazin, Pethidin, Pflanzenextrakte, Phenazon, Pheniramin, Phenylbuttersäure, Phenytoin, Phenothiazine, Phenserin, Phenylbutazon, Phenytoin, Pimecrolimus, Pimozid, Pindolol, Pioglitazon, Piperazin, Piracetam, Pirenzepin, Piribedil, Pirlindol, Piroxicam, Pitavastatin, Posaconazole, Pramipexol, Pramlintide, Pravastatin. Prazosin, Procain, Promazin, Propiverin, Propranolol, Propionsaurederivate, Propyphenazon, Prostaglandine, Protionamid, Proxyphyllin, Quetiapin Quinapril, Quinaprilat, Quinupristin. Rabeprazol, Ramipril, Ranitidin, Raloxifen, Ranolazine, Rapamycin, Rasburicase, Reboxetin, Repaclinide, Reproterol, Reserpin, Revofloxacin, Ribavirin, Rifampicin, Riluzole, Rimexolon, Risedronat, Risperidon Ritonavir, Rituximab Rivastigmin, Risatriptan, Rofecoxib, Ropinirol, Ropivacain, Rosiglitazon, Rotigotine, Roxatidin, Roxithromycin, Ruscogenin, Rosuvastatin, Rutosid und Rutosidderivate, Sabadilla Salbutamol, Salicylate, Salmeterol, Saperconazole, Schilddrüsenhormone, Scopolamin, Selegilin, Sertaconazol, Sertindol, Sertralin, Sevelamer, Sibutramin, Sildenafil, Silikate. Simvastatin, Sirolimus, Sitosterin. Sotalol, Spagluminsäure, Sparfloxacin, Spectinomycin, Spiramycin, Spirapril, Spironolacton, Stavudin, Streptomycin. Sucralfat, Sufentanil, Sulbactam, Sulfonamide, Sulfasalazin, Sulpirid, Sultamicillin, Sultiam, Sumatriptan, Suxamethoniumchlorid, Tacrin, Tacrolimus, Tadalafil, Taliolol, Talsaclidin, Tamoxifen, Tamsulosin, Tasonermin, Tazaroten, Tegafur, Tegaserod, Telithromycin, Telmisartan, Temoporfin Temozolomid, Tenatoprazol, Tenecteplase, Teniposid, Tenofovir, Tenoxicam, Teriparatid, Terazosin, Terbinafin, Terbutalin, Terfenadin, Teriparatid, Terlipressin, Tertatolol, Testosteron und Testosteronderivate, Tetracycline, Tetryzolin, Tezosentan, Theobromin, Theophyllin, Theophyllinderivate. Thiamazol, Thiamphenicol, Thiotepa, Thr. Wachstumsfaktoren, Tiagabin, Tiaprid, Tibolon, Ticlopidin, Tilidin. Timolol, Tinidazol, Tioconazol, Tioguanin, Tiotropium, Tioxolon, Tirazetam, Tiropramid, Trofiban, Tizanidin, Tolazolin, Tolbutamid Tolcapon, Tolnaftat, Tolperison, Tolterodin, Topiramat, Topotecan, Torasemid, Tramadol, Tramazolin, Trandolapril, Tranylcypromin, Trapidil, Trastuzumab, Travoprost, Trazodon, Trepostinil, Triamcinolon und Triamcinolonderivate, Tnamteren, Triflupendol, Trifluridin Trimetazidine, Trimethoprim, Trimipramin, Tripelennamin, Triprolidin , Trifosfamid, Tromantadin, Trometamol, Tropalpin, Trovafloxacin, Troxerutin, Tulobuterol, Trypsine, Tyramin, Tyrothricin, Urapidil, Ursodeoxycholsäure, Theophyllin Ursodeoxycholsäure, Valaciclovir, Valdecoxib, Valganciclovir, Valproinsäure, Valsartan, Vancomycin, Vardenafil, Vecuroniumchlorid Venlafaxin, Verapamil, Verteporfin, Vidarabin, Vigabatrin, Viloxazin, Vinblastin, Vincamin, Vincristin, Vindesin, Vinorelbin, Vinpocetin, Viquidil, Vitamin D und Denvate von Vitamin D Voriconazol, Warfarin, Xantinolnicotinat, Ximelagatran, Xipamid Zafirlukast, Zalcitabin, Zaleplon, Zanamivir, Zidovudin, Ziprasidon, Zoledronsäure, Zolmitriptan, Zolpidem, Zoplicon, Zotepin und dergleichen.

Die Wirkstoffe können gewünschtenfalls auch in Form ihrer pharmazeutisch annehmbaren Salze oder Derivate verwendet werden, und im Falle chiraler Wirkstoffe können sowohl optisch aktive Isomere als auch Racemate oder Diastereoisomerengemische eingesetzt werden. Gewünschtenfalls können die erfindungsgemäßen Zusammensetzungen auch zwei oder mehrere pharmazeutische Wirkstoffe enthalten.

### Peptid- oder Protein-Wirkstoffe

Die erfindungsgemäße Arzneiform eignet sich bevorzugt für Peptid- oder Protein-Wirkstoffe, die mit einer dem Wirkstoff zugeordnete, die Verabreichung des Wirkstoffs fördernde Subtanz formuliert sind. Geeignete Formulierungen sind z. B. aus der WO 2005/007139 bekannt.

### Peptidwirkstoffe mit einem Molekuargewicht Mw < 3.000

Abarelix, Angiotensin II, Anidulafungin, Antide, Argipressin, Azalin und Azalin B, Bombesin-Antagonist, Bradykinin, Buserelin, Cetrorelix, Ciclosporin A, Desmopressin, Detirelix, Enkephaline (Leu-, Met-) Ganirelix, Gonadorelin, Goserelin, Growthhormone-Secretagogue, Micafungin, Nafarelin, Leuprolide, Leuprorelin, Octreotid, Orntide, Oxytocin, Ramorelix, Sekretin, Somatotropin, Terlipressin, Tetracosactid, Teverelix, Triptorelin, Thyroliberin, Thyrotropin oder Vasopressin

### Protein oder Peptid-Wirkstoffe mit einem mittleren Molekulargewicht M_{w} von 3.000 bis 10.000

Calcitonin, Corticotropin, Endorphine, Epithelial growth factor, Glucagon, Insulin, Novolin, Parathyroid Hormon, Relaxin, Pro-Somatostatin oder Salmon Secretin ist.

### Protein oder Peptid-Wirkstoffe mit einem mittleren Molekulargewicht M_{w} von 3.000 bis 10.000

Interferon (alpha, beta, gamma), Interleukine (IL1, IL2), Somatotropin, Erytropoietin, Tumornekrosefaktor (TNF alpha, beta), Relaxin, Endorphin, Dornase alpha, Folikel stimulierendes Hormon (FSH), Human Chorion Gonadotropin (HCG), Human Growth Hormone Release factor (hGRF), Luteinisierendes Hormon (LH) oder Epidermal Growth Factor

### Nukleinsäure-Wirkstoffe

Die erfindungsgemäße Arzneiform eignet sich bevorzugt für Nukleinsäure-Wirkstoffe, die mit einer dem Wirkstoff zugeordnete, die Verabreichung des Wirkstoffs fördernde Subtanz formuliert sind. Geeignete Formulierungen sind z. B. aus der WO 2006/061069 bekannt.

Nukleinsäure-Wirkstoffe haben in der Regel die Aufgabe, am Zielort in-vivo eine Wechselwirkung mit der DNA von Säugerzellen, insbesondere menschlichen Zellen hervorzurufen, die zu einer veränderten DNA-Struktur in der Zelle oder ganz allgemein zu veränderten Zelleigenschaften führen. In erster Linie ist hier die sogenannte Gentherapie zu nennen, deren Ziel die Reparatur defekter Genstrukturen bei genetisch bedingten Erkrankungen ist. Hierbei kann es sich z. B. um eine Inaktivierung oder ein Abschalten unerwünschter Genaktivitäten handeln, wie z. B. der Telomeraseaktivität in Tumorzellen. Es kann sich auch um ein Wiederherstellen üblicherweise in gesunden Zellen vorhandener Genaktivitäten handeln, z. B. der p53-Genaktivität, einem seit lange bekannten, intensiv beforschten Tumorsuppressor-Gen. Die Erfindung betrifft demnach oral verabreichbare Arzneiformen für Nukleinsäure-Wirkstoffe, insbesondere für Gentherapie.

Der Nukleinsäure-Wirkstoff kann eine einzel- oder doppelsträngige DNA (Desoxyribonukleinsäure) oder RNA (Ribonukleinsäure) oder ein DNA-RNA-Chimär sein, wobei natürlich vorkommende und/oder nicht natürlich vorkommende synthetisch modifizierte Nukleotide enthalten sein können. Der Nukleinsäure-Wirkstoff kann linear oder ringförmig vorliegen. Es kann sich um Oligonukleotideinheiten, z. B. mit einer Länge von 10 bis 200 Basen oder Basenpaaren handeln. Es kann sich auch um längere Einheiten von z. B. über 200 bis 100.000, 500 bis 10.000 oder 1000 bis 5.000 Basen oder Basenpaaren handeln. Neben der als eigentlichem Wirkstoff fungierenden Sequenz, z. B. einer Nukleinsäuresequenz die in der Zielzelle vorhanden ist oder ergänzt werden soll, können im Nukleinsäure-Wirkstoff gegebenenfalls auch Vektor-Sequenzen enthalten sein, die in der Regel nicht in der Zielzelle vorhanden sind und nicht mit dieser wechselwirken sollen.

Bekannt sind z. B. auf doppelsträngiger DNA basierende Vektorsysteme, die auf Plasmiden oder auf viralen Systemen basierenden Vektoren beruhen. Bekannt sind z. B. rekombinante Adeno-assoziierte-virale Vektoren (rAAV). Andere doppelsträngige Vektoren können Promotor- oder Regulationssequenzen aus Cytomegalieviren (CMV) oder dem SV40-Virus enthalten. Andere Vektoren können auf einzelsträngiger DNA beruhen, die mit Hilfe von angefügten RNA Elementen gegenüber Abbau geschützt werden kann. Bekannt sind auch sogenannte RDO I und RDO II Konstrukte, bei denen kurze DNA-Stücke, z. B. 30 bis 60 Basen an den Enden mit kurzen RNA Stücken von 1 bis 4 Basen versehen werden. Zur zusätzlichen Erhöhung der Halbwertszeit bzw. der Nukleaseresistenz können nicht natürlich vorkommende Nukleotide in die RNA oder DNA eingefügt werden. Dabei können z. B. einzelne Sauerstoffatome durch Schwefelatome ersetzt sein, so dass man Phosphor-Schwefel-Brücken erhält (MSO). Die Vielfalt als Gene-Repair oder Gene-Replacement-Vektoren geeigneten Nukleinsäure Formen, die als Wirkstoffe im Sinne der vorliegenden Erfindung eingesetzt werden können, ist z. B. Nature Reviews Vol.4, 2003, S. 679 - 689, Li Liu *et al.* beschrieben. Bevorzugt sind Nukleinsäure-Fragmente, die im Wesentlichen nur die als Wirkstoff fungierende Nukleinsäure-Sequenz und keine oder nur geringe Anteile an Vektor-DNA enthalten.

Der Nukleinsäure-Wirkstoff kann in einem Komplex oder Konjugat, z. B. mit kationischen Polymeren oder Proteinen wie z. B. Antikörpern, vorliegen. Die Komplexierung bzw. Konjugat-Bindung kann reversibel oder irreversibel kovalent durch chemische Brückenbindung oder nebenvalent über Van der Waals-Kräfte, ionische Bindungen, hydrophobe Bindung geschehen. Die neben dem Nukleinsäure-Wirkstoff im Komplex- oder Konjugat entfaltenen Moleküle entfalten jedoch selbst keine therapeutische Wirkung und sind somit als Formulierungshilfsmittel und nicht als Wirkstoff oder Teil des Wirkstoffs anzusehen.

Der Nukleinsäure-Wirkstoff kann gegebenenfalls unter Zuhilfenahme von Proteinen oder Peptiden formuliert werden. Diese entfalten jedoch selbst jedoch keine therapeutische Wirkung und sind somit als Formulierungshilfsmittel und nicht als Wirkstoff oder Teil des Wirkstoffs anzusehen.

Die Nukleinsäure kann z. B. gemäß der WO 02/094983 in Form eines Komplexes mit einem Antikörper, der spezifisch an die Nukleinsäure bindet, und einer kationischen Substanz vorliegt. Es konnte gezeigt werden, dass diese Maßnahme zu einer erhöhten Transfektionsrate sowohl in-vitro als auch in-vivo beitragen kann. Es kann sich dabei bevorzugt um monoklonale IgG-Antikörper oder IgM-Antikörper handeln, die vollständig oder auch als Fragmente, Fc-Antikörper-Fragmente, Fab'-Antikörper-Fragmente, F(a,b)'2-Antikörperfragmente oder halbe Antikörperfragmente handeln, die aber jeweils mindestens eine Anti-DNA-Bindungsstelle enthalten müssen. Das molekulare Verhältnis von Nukleinsäure zu Anti-DNA-Antikörper kann z. B. 1 zu 20 bis 1 zu 5 betragen.

Der Nukleinsäure-Wirkstoff kann z. B. die Therapie der Hämophilie zum Ziel haben und ein Blutgerinnungsfaktor-Gen, z. B. das cDNA-Gen des menschlichen Blutgerinnungsfaktors IX enthalten (s. z. B. WO 03/028657 oder Palmer et al., Blood, 1989, 73(2), p.438-445 oder Yao et al., Proc Natl Acad Sci U S A, 1992, 89(8): p.3357-3361). Neben dem therapeutisch wirksamen Genanteil kann der Nukleinsäure-Wirkstoff auch ein Immuntoleranzinduzierendes Gen, wie z. B. den *Fas*-Ligand enthalten. Der coexprimierte *Fas-*Ligand bzw. -Genabschnitt kann die Apoptose in T-Zellen einleiten, die nach dem Gentransfer in die Zielzellen spezifisch aktiviert werden können. Vektoren in Zusammenhang mit der Apoptose-Induktion in Leukämie-Zellen sind auch aus Walensky et al., 2004. "Activation of Apoptosis in Vivo by a Hydrocarbon-Stapled BH3 Helix", Science, 305, S. 1466 - 1470 abzuleiten.

Der Nukleinsäure-Wirkstoff kann z. B. einen Genabschnitt, insbesondere die Promotorregion, des humanen Telomerase-Gens enthalten. Geeignet ist z. B. der in WO 99/38964 beschriebene Gentherapie-Vektor pGT62-codAupp oder andere für einen Fachmann aus der WO 99/38964 ableitbare Vektoren. Der Nukleinsäure-Wirkstoff kann ein Tumorsuppressor-Genabschnitt enthalten, z. B. das p53-Tumorsuppressor-Gen oder Fragmente davon. US 6,451,593 B1 beschreibt Konstruktionsprinzipien für Expressionsvektoren für die Gentherapie, die zur Herstellung von Nukleinsäure-Wirkstoffen in Sinne der Erfindung geeignet sind.

### BEISPIELE

### Teil A) Wirkungen der Teilneutralisation mit Lysin

### Freisetzungstest der Theophyllin-Pellets nach USP 28 <711> Paddle-Methode (= Apparatus 2)

Ablauf:
1. Die Gefäße der Freisetzungsaparatur werden mit je 360 ml 0,1M-HCl (ph 1,2) gefüllt und die Temperatur des Wasserbads auf 37 ± 0,5°C eingestellt.
2. Der Blattrührer wird mit einer Umdrehungsrate von 100 Upm angeschaltet.
3. In jedes Gefäß der Apparatur werden 1g Pellets gegeben. Es wird darauf geachtet, dass keine Luftblasen auf der Pellet-Oberfläche sind.
4. Nach 120 min werden 140 ml Phosphat-Pufferlösung (auf 37°C temperiert) zugesetzt, so dass im Endvolumen von 500 ml der gewünschte pH-Wert entsteht: pH 5,5; 5,6; 5,7; 5,8 oder 7,0.
5. Bestimmung des Zeitpunktes der 100%-igen Wirkstofffreigabe (photometrisch bei 271nm, im Umlaufverfahren). Ergebnisse siehe Tabelle 1.
Tabelle 1

| | Theophyllin Pellets mit 30%-igen Überzug aus einem Copolymer aus 50 Gew.-% Ethylacrylat und 50 Gew.-% Methacrylsäure (EUDRAGIT® L 30 D-55), | | | | | |
|---|---|---|---|---|---|---|
| | 90 % Wirkstoff freigesetzt [min], Verfahren nach USP 28 Paddle, | | | | | |
| | Beispiel A1 | | Beispiel A2 | | Beispiel A3 | |
| | 15 %Teilneutralisation mit Lysin | | 15 %Teilneutralisation mit NaOH | | keine Teilneutralisation | |
| vorher 2h pH 1,2 | + | - | + | - | + | - |
| pH 5,5 | 45 | 40 | 90 | 45 | 120 | 120 |
| pH 5,6 | 30 | 28 | 50 | 30 | 60 | 60 |
| pH 5,7 | 20 | 19 | 30 | 20 | 50 | 50 |
| pH 5,8 | 18 | 17 | 20 | 18 | 30 | 30 |
| pH 7,0 | Sofortige Wirkstofffreisetzung | | | | | |

### Beispiel A1. Rezeptur mit Lysin

Pelletüberzüge mit EUDRAGIT L 30 D 55 (30%-ige Dispersion, enthaltend ein Copolymer aus 50 Gew.-% Ethylacrylat und 50 Gew.-% Methacrylsäure) teilneutralisiert mit Lysin. Auf 100 g Theophyllin Pellets, der Firma Klinge Pharma, mit der Teilchengröße von 0,7 -1,0 mm werden 30 % Trockensubstanz einer Polymerdispersispersion (Methacrylatcopolymer aus 50 Gew. - % Methacrylsäure und 50 Gew. - % Ethylacrylat) mit folgenden Rezepturen überzogen. Der Gesamttrockengehalt -Auftrag beträgt 35,7 Gew. % bezogen auf Ansatzmenge

Freigabeuntersuchung 90 Gew.- % des Wirkstoffes siehe Tabelle 1.

| Materialien | (g) |
|---|---|
| EUDRAGIT^{®} L 30 D-55 | 100,00 |
| Lysin | 3,69 |
| Glycerinmonostearat | 1,50 |
| Polysorbat 80 | 0,60 |
| Wasser dem. | 132,81 |
| Summe | 238,60 |

Sprühparameter im Hüttlin Mycrolab:

| | |
|---|---|
| Sprühdüse | 0,6 mm |
| Sprührate | 26 g/min/kg |
| Sprühdruck | 1,0 bar |
| Microklima | 0,6 bar |
| Zuluft Airflow | 20 m³ |
| Zulufttemperatur | 33-39 °C |
| Produkttemperatur | 26-29 °C |
| Machtrocknungszeit im Gerät | 10 min bei 40 °C |
| Sprühzeit | 1,5-2 h |
| Trocknung über Nacht bei | Raumtemperatur (RT) |

### Beispiel A2. Rezeptur mit NaOH

Pelletüberzug mit EUDRAGIT® L 30 D 55 teilneutralisiert mit NaOH. Auf 100 g Theophyllin Pellets, der Firma Klinge Pharma, mit der Teilchengröße von 0,7 -1,0 mm werden 30 % Trockensubstanz einer Polymerdispersispersion (Methacrylatcopolymer aus 50 Gew. - % Methacrylsäure und 50 Gew. - % Ethylacrylat) mit folgenden Rezepturen überzogen. Der Gesamttrockengehalt - Auftrag beträgt 33,11 Gew. % bezogen auf Ansatzmenge Freigabeuntersuchung 90 Gew.- % des Wirkstoffes siehe Tabelle 1.

| Materialien | (g) |
|---|---|
| EUDRAGIT^{®} L 30 D-55 | 100,00 |
| NaOH | 1,01 |
| Glycerin monostearat | 1,50 |
| Polysorbat 80 | 0,60 |
| Wasser dem. | 117,62 |
| Summe | 220,73 |

Sprühparameter im Hüttlin Mycrolab:

| | |
|---|---|
| Sprühdüse | 0,6mm |
| Sprührate | 27g/min/kg |
| Sprühdruck | 1,0 bar |
| Microklima | 0,6 bar |
| Zuluft Airflow | 20 m³ |
| Zulufttemperatur | 33 - 40 °C |
| Produkttemperatur | 26 - 30 °C |
| Machtrocknungszeit im Gerät | 10 min bei 40 °C |
| Sprühzeit | 1 - 1,5 h |

Trocknung über Nacht bei RaumtemperaturT

### Beispiel A3. Rezeptur ohne Teilneutralisierung

Pelletüberzug mit EUDRAGIT L 30 D 55 ohne Teilneutralisierung. Auf 100 g Theophyllin Pellets, der Firma Klinge Pharma, mit der Teilchengröße von 0,7 -1,0 mm werden 30 % Trockensubstanz einer Polymerdispersispersion (Methacrylatcopolymer aus 50 Gew. - % Methacrylsäure und 50 Gew. - % Ethylacrylat) mit folgenden Rezepturen überzogen. Der Gesamttrockengehalt - Auftrag beträgt 32,111 Gew. % bezogen auf Ansatzmenge. Freigabeuntersuchung 90 Gew.- % des Wirkstoffes siehe Tabelle 1.

| Materialien | (g) |
|---|---|
| EUDRAGIT^{®} L 30 D-55 | 100,00 |
| Glycerin monostearat | 1,50 |
| Polysorbat 80 | 0,60 |
| Wasser dem. | 136,83 |
| Summe | 238,93 |

Sprühparameter im MiniGlatt:

| | |
|---|---|
| Sprühdüse | 0,5 mm |
| Sprührate | 1 - 2 g/min |
| Sprühdruck | 0,8 bar |
| Zuluft | 0,7 bar |
| Zulufttemperatur | 35 - 37 °C |
| Produkttemperatur | 32 - 33 °C |
| Machtrocknungszeit im Gerät | 10 min bei 40 °C |
| Sprühzeit | ca. 2 - 3 h |
| Trocknung über Nacht bei | Raumtemperatur |

### Teil B) Arzneiformen mit dreischichtiger Trennschicht und gegebenenfalls mit Lysin teilneutralisierten Polymerüberzügen

### Herstellung der Überzüge

Für alle Überzüge wurden als Ausgangsmaterial Theophyllin - Pellets 710 - 1250 µm der Fa. Klinge Pharm mit 94,13% Theophyllingehalt eingesetzt

### Beispiel B1 (Vergleich, nicht erfindungsgemäß)

### Standard EUDRAGIT^{®} L 30 D-55 Formulierung:

Auf 100 g Theophyllin Pellets, 710 -850 µm werden mit einer wässrigen Sprühsuspension 15,0 %-ig an Trockensubstanz im Hüttlin Mycrolab aufgesprüht. Zusammensetzung der Suspension: 66,7 g EUDRAGIT^{®} L 30 D-55, 2,0 g Triethylcitrat, 1,5 g Glycerinmonostearat, 0,6 g Polysorbat 80. Die Wirkstofffreigabe, dieser Pellets nach USP Nr 2 (Paddle) bzw Restwirkstoffgehalt nach 2 Stunden in 0,1 N HCl und anschließend Phosphatpuffer pH 5,8 beträgt:

| Zeit | 120 min | 140 min | 145 min |
|---|---|---|---|
| Schichtdicke MW gesamt nach Magensaft [µm] | 29 -38 | 18 -25 | 0 - 9 |
| Restwirkstoff in Formulierung [%] | 99,5 | 90,5 | 78,3 |

### Beispiel B2 (Vergleich, nicht erfindungsgemäß)

Formulierung mit EUDRAGIT^{®} L 30 D-55 und Lysin 15%-ig teilneutralisiert: Auf 100 g Theophyllin Pellets, 710 -850 µm werden mit einer wässrigen Sprühsuspension 15,0 % ig an Trockensubstanz im Hüttlin Mycrolab, aufgesprüht. Zusammensetzung der Suspension: 66,7 g EUDRAGIT^{®} L 30 D-55, 2,5 g Lysin, 2,0 g Triethylcitrat, 1,5 g Glycerinmonostearat, 0,6 g Polysorbat 80. Die Wirkstofffreigabe bzw. Restwirkstoffgehalt dieser Pellets nach USP Nr. 2 (Paddle) 2 Stunden in 0,1 N HCl und anschließend Phosphatpuffer pH 5,8 beträgt:

| Zeit | 120 min | 140 min | 145 min |
|---|---|---|---|
| Schichtdicke MW gesamt nach Magensaft [µm] | 18 -38 | 19 - 33 | 9 - 35 |
| Restwirkstoff in Formulierung [%] | 93,7 | 71,0 | 56,4 |

### Beispiel B3 (Vergleich, nicht erfindungsgemäß)

Formulierung mit EUDRAGIT^{®} L 30 D-55 und HPMC als einschichtige Trennschicht:
A.) 10,0 g HPMC (Methocel E 5) in 132,9 g dem. Wassergelöst. Auf 100 g Theophyllin Pellets, 710 -850 µm wird im Hüttlin Mycrolab ein Sprühauftrag durchgeführt.

Anschließend wird auf 100 g der Pellets von A.) die Sprühsuspension von Beispiel 1 aufgesprüht

Die Wirkstofffreigabe bzw. Restwirkstoffgehalt dieser Pellets nach USP Nr. 2 (Paddle) 2 Stunden in 0,1 N HCl und anschließend Phosphatpuffer pH 5,8 beträgt:

| Zeit | 120 min | 140 min | 145 min |
|---|---|---|---|
| Schichtdicke MW gesamt nach Magensaft [µm] | 24 - 35 | 8 - 15 | 0 |
| Restwirkstoff in Formulierung [%] | 99,4 | 81,0 | 36,6 |

### Beispiel B4 (erfindungsgemäß)

Formulierung mit EUDRAGIT^{®} L 30 D-55 und HPMC/ Caprinsäure/ HPMC als dreischichtige Trennschicht:
B.) 5,0 g HPMC (Methocel E 5) werden in 66,4 g dem. Wasser gelöst und auf 100 g Theophyllin Pellets, 710 -850 µm im Hüttlin Mycrolab aufgesprüht
C.) 5,0 g Caprinsäure werden in 61,7 g abs. Ethanol gelöst und auf 100,0 g Pellets von B.) ebenfalls im Hüttlin Mycrolab aufgesprüht
D.)5,0 g HPMC (Methocel E 5) werden in 66,4 g dem. Wasser gelöst und auf 100 g Pellets von C.) erneut aufgesprüht

Anschließend wird die Sprühsuspension von Beispiel 1 auf 100 g Pellets D.) aufgetragen

Die Wirkstofffreigabe bzw Restwirkstoffgehalt dieser Pellets nach USP Nr 2 (Paddle) 2 Stunden in 0.1 N HCl und anschließend Phosphatpuffer pH 5,8 beträgt:

| Zeit | 120 min | 140 min | 145 min |
|---|---|---|---|
| Schichtdicke MW gesamt nach Magensaft [µm] | 34 -51 | 4 - 19 | 0 - 12 |
| Restwirkstoff in Formulierung [%] | 99,4 | 93,6 | 80,4 |

### Beispiel B5 (nicht erfindungsgemäß)

Formulierung mit EUDRAGIT^{®} L 30 D-55 teilneutralisiert mit 15% Lysin und HPMC als einschichtige Trennschicht:
A.)10,0 g HPMC (Methocel E 5 Premium) werden in 132,9 g dem. Wasser
   gelöst anschließend auf 100 g Theophyllin Pellets, 710 -850 µm im

Hüttlin Mycrolab aufgesprüht.

Anschließend wird die Sprühsuspension von Beispiel 2 auf 100 g Pellets A.) aufgetragen

Die Wirkstofffreigabe bzw Restwirkstoffgehalt dieser Pellets nach USP Nr 2 (Paddle) 2 Stunden in 0,1 N HCl und anschließend Phosphatpuffer pH 5,8 beträgt:

| Zeit | 120 min | 140 min | 145 min |
|---|---|---|---|
| Schichtdicke MW gesamt nach Magensaft [µm] | 21 - 42 | 5 - 20 | 0 - 18 |
| Restwirkstoff in Formulierung [%] | 97,2 | 43,1 | 20,9 |

### Beispiel B6 (erfindungsgemäß)

Formulierung mit EUDRAGIT^{®} L 30 D-55 teilneutralisiert mit 15% Lysin und HPMC/ Caprinsäure/ HPMC als dreischichtige Trennschicht:
B.) 5,0 g HPMC (Methocel E 5 Premium) werden in 66,4 g dem. Wasser gelöst und auf 100 g Theophyllin Pellets, 710 -1250 µm im Hüttlin Mycrolab aufgesprüht
C.) 5,0 g Caprinsäure werden in 61,7 g abs. Ethanol gelöst und auf 100,0 g Pellets von B.) ebenfalls im Hüttlin aufgesprüht
D.)5,0 g HPMC (Methocel E 5 Premium) werden in 66,4 g dem. Wasser gelöst und auf 100 g Pellets von C.) erneut aufgesprüht

Anschließend wird die Sprühsuspension von Beispiel 2 auf 100 g Pellets D.) aufgesprüht

Die Wirkstofffreigabe bzw Restwirkstoffgehalt dieser Pellets nach USP Nr 2 (Paddle) 2 Stunden in 0,1 N HCl und anschließend Phosphatpuffer pH 5,8 beträgt:

| | 120 min | 140 min | 150 min |
|---|---|---|---|
| Schichtdicke MW gesamt nach Magensaft [µm] | 24 - 38 | 0 | 0 |
| Restwirkstoff in Formulierung [%] | 97.3 | 81,7 | 16,6 |

### Filmablösungstest.

Je 250 mg der Pellets von Beispiel B1 - B6 werden nach USP Nr. 2 (Paddle) 2 Stunden in 700 mL 0,1 N HCl gerührt, anschließend mit Na₃PO₄-Lösung auf pH 5,8 eingestellt. Die Probennahme von ca. 10 Pellets erfolgt nach 120 min (vor Umpufferung), nach 140, 145 min, bzw. nach 150 min (Phosphatpuffer pH 5,8).

Die feuchten Pellets werden auf ein saugfähiges Tuch gelegt und bei RT getrocknet. Anschließend werden Oberfläche und Bruch der Pellets unterm Raster-Elektronenmikroskop (REM) untersucht und die verbliebene Schichtdicke bestimmt.

### Bewertung des Restwirkstoffgehalts versus Schichtdicke:

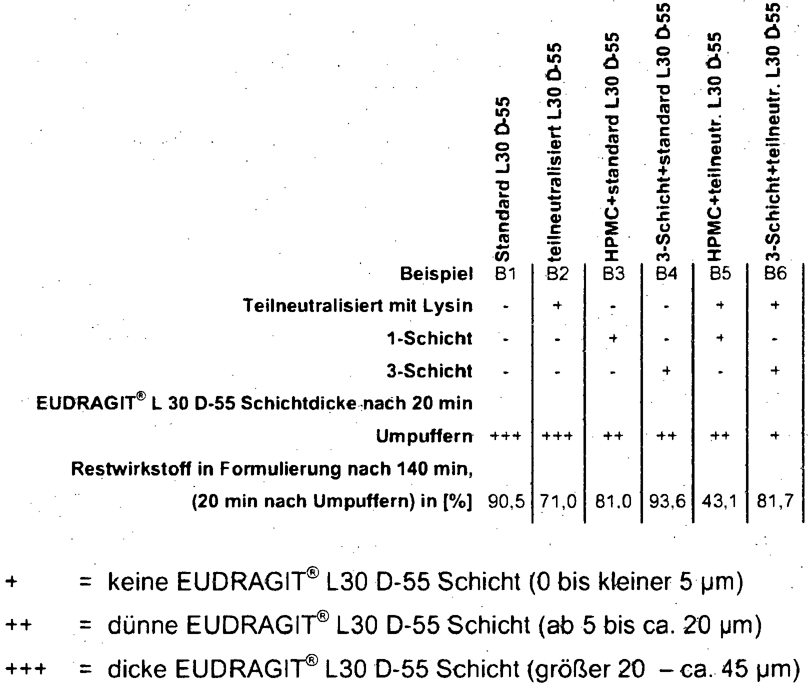

## Patentansprüche

1. Arzneiform, enthaltend einen wirkstoffhaltigen Kern, der mit einer Überzugsschicht aus einem magensaftresistenten, darmsaftlöslichen (Meth)acrylat-Copolymer umhüllt ist, wobei sich zwischen dem Kern und der Überzugsschicht eine Trennschicht, enthaltend ein filmbildendes wasserlösliches Polymer, befindet, **dadurch gekennzeichnet, dass** die Trennschicht dreischichtig ausgeführt ist, wobei eine innere Schicht mit einer wasserabweisenden Substanz vorhanden ist und zwei Schichten des filmbildenden wasserlöslichen Polymeren die Schicht mit der wasserabweisenden Substanz einschließen.

2. Arzneiform nach Anspruch 1, **dadurch gekennzeichnet, dass** das filmbildende, wasserlösliche Polymer nicht ionische Cellulosederivate, wie Hydroxypropylcellulose, Hydroxypropylmethylcellulose, sowie Natrium-Carboxymethylcellulose, Polysaccharide, wie Stärke, Amylose, Alginat, Pektin, Xanthan sowie Gelatine, Polyethylenglykole und/oder Polyvinylpyrrolidon umfasst.

3. Arzneiform nach Anspruch 1 oder 2, **dadurch gekennzeichnet, dass** die wasserabweisende Substanz ein C₈-bis C₂₄- Fettalkohol, ein Ester von C₈- bis C₂₄- Fettalkoholen mit organischen Säuren, eine C₈- bis C₂₄-Fettsäure, wie z. B. Stearinsäure oder Caprinsäure, ein Ester von C₈- bis C₂₄- Fettsäuren mit Alkoholen oder Polyalkoholen, wie z. B. Glycerolmonostearat oder Glyceroldistearat, ist.

4. Arzneiform nach einem oder mehreren der Ansprüche 1 bis **3, dadurch gekennzeichnet, dass** der magensaftresistente, darmsaftlösliche Polymerüberzug ein anionisches (Meth)acrylat-Copolymer, bestehend aus radikalisch polymerisierten Einheiten von 25 bis 95 Gew.-% C₁- bis C₄-Alkylestern der Acryl- oder der Methacrylsäure und 5 bis 75 Gew.-% (Meth)acrylat-Monomeren mit einer anionischen Gruppe, ist

5. Arzneiform nach einem oder mehreren der Ansprüche 1 bis 4, **dadurch gekennzeichnet, dass** das anionische (Meth)acrylat-Copolymer des Überzugs insgesamt oder anteilig mittels einer Base in Summe teilneutralisiert ist.

6. Arzneiform nach Anspruch 5, **dadurch gekennzeichnet, dass** als Base eine kationische, organische Base mit einem Molekulargewicht über 150 oder Lysin enthalten ist.

7. Arzneiform nach Anspruch 6, **dadurch gekennzeichnet, dass** als Base Histidin, Arginin, ein Poly-Histidin, ein Poly-Arginin, ein Poly-Lysin, ein Phopholipid, wie Phosphatidylcholin, ein Ribonukleosid oder ein Desoxyribonukleosid, eine Base aus kationischen oberflächenaktiven Hilfsstoffen oder Emulgatoren enthalten ist.

8. Arzneiform nach Anspruch 6 oder 7, **dadurch gekennzeichnet, dass** der Polymerüberzug Lysin und/oder Arginin in Kombination mit 5 bis 25 Gew.-% eines Weichmachers bezogen auf das Polymer enthält.

9. Arzneiform nach Anspruch 6 bis 8, **dadurch gekennzeichnet, dass** der Polymerüberzug Lysin und/oder Arginin in einer Konzentration von 10 bis 30 Gew.-% bezogen auf das Polymer enthält.

10. Arzneiform, nach einem oder mehreren der Ansprüche 1 bis 9, **dadurch gekennzeichnet, dass** der Kern einen Wirkstoff und eine dem Wirkstoff zugeordnete, die Verabreichung des Wirkstoffs fördernde Substanz enthält.

11. Arzneiform nach Anspruch 10, **dadurch gekennzeichnet, dass** der Wirkstoffe ein Peptid, ein Protein, eine Nukleinsäure oder ein Polysaccharid oder ein Derivat der genannten Stoffklassen ist.

12. Arzneiform, nach Anspruch 10 oder 11, **dadurch gekennzeichnet, dass** die dem Wirkstoff zugeordnete, die Verabreichung des Wirkstoffs fördernde Subtanz ein Penetrationsförderer und/oder oder ein mucoadhaesives Polymer und/oder eine Substanz ist, die den enzymatischen Abbau des Wirkstoffs durch in Verdauungstrakt vorkommende Enzyme hemmt, oder ein Efflux-Pumpen-Inhibitor (Pgp-Inhibitor) ist.

13. Verfahren zur Herstellung einer Arzneiform nach einem oder mehreren der Ansprüche 1 bis 12 in an sich bekannter Weise mittels pharmazeutisch üblicher Verfahren, wie direktem Verpressen, Verpressen von Trocken-, Feucht- oder Sintergranulaten, Extrusion und anschließende Ausrundung, feuchte oder trockene Granulation oder direkte Pelletierung oder durch Binden von Pulvern (Powder layering), durch Aufsprühen von Suspensionen oder Lösungen auf wirkstofffreie Kugeln bzw. neutrale Kerne (Nonpareilles) oder wirkstoffhaltige Partikeln und mittels Auftrag des Polymerüberzugs im Sprühverfahren oder durch Wirbelschichtgranulation.

14. Verwendung einer Arzneiform mit einer dreischichtigen Trennschicht nach einem oder mehreren der Ansprüche 1 bis 12 zur Formulierung von Arzneiformen für Protein-, Peptid, Nukleinsäure- oder Polysaccharid Wirkstoffen oder Derivaten davon.

## Claims

1. Pharmaceutical form, comprising an active compound-containing core, which is covered with a coating layer of a gastric juice-resistant, intestinal juice-soluble (meth)acrylate copolymer, where between the core and the coating layer is situated a separating layer, comprising a film-forming water-soluble polymer, **characterized in that** the separating layer is designed to be three-layer, wherein an inner layer containing a water-repellent substance is present and two layers of the film-forming water-soluble polymer enclose the layer containing the water-repellent substance.

2. Pharmaceutical form according to Claim 1, **characterized in that** the film-forming, water-soluble polymer comprises non-ionic cellulose derivatives, such as hydroxypropylcellulose, hydroxypropylmethyl-cellulose, and sodium carboxymethylcellulose, polysaccharides, such as starch, amylose, alginate, pectin, xanthan and gelatins, polyethylene glycols and/or polyvinylpyrrolidone.

3. Pharmaceutical form according to Claim 1 or 2, **characterized in that** the water-repellent substance is a C₈- to C₂₄-fatty alcohol, an ester of C₈- to C₂₄-fatty alcohols with organic acids, a C₈- to C₂₄-fatty acid, such as, for example, stearic acid or capric acid, an ester of C₈- to C₂₄-fatty acids with alcohols or polyalcohols, such as, for example, glycerol monostearate or glycerol distearate.

4. Pharmaceutical form according to one or more of Claims 1 to 3, **characterized in that** the gastric juice-resistant, intestinal juice-soluble polymer coating is an anionic (meth)acrylate copolymer, consisting of free radical-polymerized units of 25 to 95% by weight of C₁- to C₄-alkyl esters of acrylic or of methacrylic acid and 5 to 75% by weight of (meth)acrylate monomers having an anionic group.

5. Pharmaceutical form according to one or more of Claims 1 to 4, **characterized in that** the anionic (meth)acrylate copolymer of the coating is entirely or proportionately partially neutralized in total by means of a base.

6. Pharmaceutical form according to Claim 5, **characterized in that** the base contained is a cationic, organic base having a molecular weight of over 150 or lysine.

7. Pharmaceutical form according to Claim 6, **characterized in that** the base contained is histidine, arginine, a polyhistidine, a polyarginine, a polylysine, a phospholipid, such as phosphatidylcholine, a ribonucleoside or a deoxyribonucleoside, a base of cationic surface-active excipients or emulsifiers.

8. Pharmaceutical form according to Claim 6 or 7, **characterized in that** the polymer coating contains lysine and/or arginine in combination with 5 to 25% by weight of a plasticizer based on the polymer.

9. Pharmaceutical form according to Claim 6 to 8, **characterized in that** the polymer coating contains lysine and/or arginine in a concentration of 10 to 30% by weight based on the polymer.

10. Pharmaceutical form, according to one or more of Claims 1 to 9, **characterized in that** the core contains an active compound and a substance assigned to the active compound, promoting the administration of the active compound.

11. Pharmaceutical form according to Claim 10, **characterized in that** the active compound is a peptide, a protein, a nucleic acid or a polysaccharide or a derivative of the substance classes mentioned.

12. Pharmaceutical form, according to Claim 10 or 11, **characterized in that** the substance assigned to the active compound, promoting the administration of the active compound, is a penetration promoter and/or a mucoadhesive polymer and/or a substance which inhibits the enzymatic degradation of the active compound by enzymes occurring in the digestive tract, or an efflux pump inhibitor (Pgp inhibitor).

13. Process for the production of a pharmaceutical form according to one or more of Claims 1 to 12 in a manner known per se by means of pharmaceutically customary processes, such as direct compression, compression of dry, moist or sintered granules, extrusion and subsequent rounding, moist or dry granulation or direct pelleting or by binding of powders (powder layering), by spraying suspensions or solutions onto active compound-free beads or neutral cores (nonpareils) or active compound-containing particles and by means of application of the polymer coating in the spray process or by fluidized bed granulation.

14. Use of a pharmaceutical form having a three-layer separating layer according to one or more of Claims 1 to 12 for the formulation of pharmaceutical forms for protein, peptide, nucleic acid or polysaccharide active compounds or derivatives thereof.

## Revendications

1. Forme médicamenteuse, contenant un noyau contenant un agent actif, qui est enrobé avec une couche de revêtement en un copolymère de (méth)acrylate résistant au suc gastrique et soluble dans le chyle, une couche de séparation contenant un polymère filmogène soluble dans l'eau se trouvant entre le noyau et la couche de revêtement, **caractérisée en ce que** la couche de séparation est configurée à trois couches, une couche intérieure munie d'une substance hydrophobe étant présente et deux couches du polymère filmogène soluble dans l'eau entourant la couche munie de la substance hydrophobe.

2. Forme médicamenteuse selon la revendication 1, **caractérisée en ce que** le polymère filmogène soluble dans l'eau comprend les dérivés de cellulose non ioniques, tels que l'hydroxypropylcellulose, l'hydroxypropylméthylcellulose, ainsi que la carboxyméthylcellulose de sodium, les polysaccharides, tels que l'amidon, l'amylose, l'alginate, la pectine, le xanthane, ainsi que les gélatines, les polyéthylène glycols et/ou la polyvinylpyrrolidone.

3. Forme médicamenteuse selon la revendication 1 ou 2, **caractérisée en ce que** la substance hydrophobe est un alcool gras en C₈ à C₂₄, un ester d'alcools gras en C₈ à C₂₄ avec des acides organiques, un acide gras en C₈ à C₂₄, tel que p. ex. l'acide stéarique ou l'acide caprique, un ester d'acides gras en C₈ à C₂₄ avec des alcools ou des polyalcools, tel que p. ex. le monostéarate de glycérol ou le distéarate de glycérol.

4. Forme médicamenteuse selon une ou plusieurs des revendications 1 à 3, **caractérisée en ce que** le revêtement polymère résistant au suc gastrique et soluble dans le chyle est un copolymère de (méth)acrylate anionique, constitué par des unités polymérisées par voie radicalaire de 25 à 95 % en poids d'esters alkyliques en C₁ à C₄ de l'acide acrylique ou méthacrylique et 5 à 75 % en poids de monomères de (méth)acrylate contenant un groupe anionique.

5. Forme médicamenteuse selon une ou plusieurs des revendications 1 à 4, **caractérisée en ce que** le copolymère de (méth)acrylate anionique du revêtement est au total partiellement neutralisé en totalité ou proportionnellement avec une base.

6. Forme médicamenteuse selon la revendication 5, **caractérisée en ce qu'**une base organique cationique d'un poids moléculaire supérieur à 150 ou de la lysine est contenue en tant que base.

7. Forme médicamenteuse selon la revendication 6, **caractérisée en ce que** de l'histidine, de l'arginine, une poly-histidine, une poly-arginine, une poly-lysine, un phospholipide, tel que la phosphatidylcholine, un ribonucléoside ou un désoxyribonucléoside, une base constituée d'adjuvants ou d'émulsifiants tensioactifs cationiques est contenu en tant que base.

8. Forme médicamenteuse selon la revendication 6 ou 7, **caractérisée en ce que** le revêtement polymère contient de la lysine et/ou de l'arginine en combinaison avec 5 à 25 % en poids d'un plastifiant, par rapport au polymère.

9. Forme médicamenteuse selon les revendications 6 à 8, **caractérisée en ce que** le revêtement polymère contient de la lysine et/ou de l'arginine en une concentration de 10 à 30 % en poids, par rapport au polymère.

10. Forme médicamenteuse selon une ou plusieurs des revendications 1 à 9, **caractérisée en ce que** le noyau contient un agent actif et une substance attribuée à l'agent actif, facilitant l'administration de l'agent actif.

11. Forme médicamenteuse selon la revendication 10, **caractérisée en ce que** l'agent actif est un peptide, une protéine, un acide nucléique ou un polysaccharide ou un dérivé des classes de substances citées.

12. Forme médicamenteuse selon la revendication 10 ou 11, **caractérisée en ce que** la substance attribuée à l'agent actif, facilitant l'administration de l'agent actif, est un promoteur de pénétration et/ou un polymère muco-adhésif et/ou une substance inhibant la décomposition enzymatique de l'agent actif par les enzymes présentes dans le système digestif ou un inhibiteur de pompes d'efflux (inhibiteur de Pgp).

13. Procédé de fabrication d'une forme médicamenteuse selon une ou plusieurs des revendications 1 à 12 d'une manière connue en soi par des procédés pharmaceutiques usuels, tels que la compression directe, la compression de granulats secs, humides ou frittés, l'extrusion et l'arrondissement ultérieur, la granulation sèche ou humide ou le pastillage direct ou par liaison de poudres (« powder layering »), par pulvérisation de suspensions ou de solutions sur des billes sans agent actif ou des noyaux neutres (« nonpareilles ») ou des particules contenant un agent actif, et par application du revêtement polymère par un procédé de pulvérisation ou par granulation en lit fluidisé.

14. Utilisation d'une forme médicamenteuse comprenant une couche de séparation tricouche selon une ou plusieurs des revendications 1 à 12 pour la formulation de formes médicamenteuses pour agents actifs protéines, peptides, acides nucléiques ou polysaccharides ou leurs dérivés.
